(19) Europäisches Patentamt / European Patent Office / Office européen des brevets

(11) **EP 3 502 234 B1**

(12) **EUROPEAN PATENT SPECIFICATION**

(45) Date of publication and mention of the grant of the patent:
**30.04.2025  Bulletin 2025/18**

(51) International Patent Classification (IPC):
**C12N 1/20** (2006.01)    **C12M 1/00** (2006.01)
**C02F 3/28** (2023.01)

(21) Application number: **17210274.1**

(22) Date of filing: **22.12.2017**

(52) Cooperative Patent Classification (CPC):
**C12N 1/20; C02F 3/307; C12M 21/00;** C02F 3/341;
C02F 3/348

(54) **ANAEROBIC AMMONIUM OXIDATION WITH PLANKTONIC CELLS IN A CONTINUOUS OR SEMI-CONTINUOUS STIRRED-TANK REACTOR**

ANAEROBE AMMONIUMOXIDATION MIT PLANKTONISCHEN ZELLEN IN EINEM KONTINUIERLICHEN ODER HALBKONTINUIERLICHEN RÜHRKESSELREAKTOR

OXYDATION ANAÉROBIE D'AMMONIUM À L'AIDE DE CELLULES PLANCTONIQUES DANS UN RÉACTEUR À RÉSERVOIR À AGITATION CONTINUE OU SEMI-CONTINUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.06.2019  Bulletin 2019/26**

(73) Proprietor: **Helmholtz-Zentrum für Umweltforschung GmbH-UFZ**
**04318 Leipzig (DE)**

(72) Inventors:
• **ADRIAN, Lorenz**
  **10407 Berlin (DE)**
• **DING, Chang**
  **04105 Leipzig (DE)**

(74) Representative: **Patentanwälte Bressel und Partner mbB**
**Potsdamer Platz 10**
**10785 Berlin (DE)**

(56) References cited:
• **ZHIGANG LI ET AL: "Factors affecting the treatment of reject water by the anammox process", BIORESOURCE TECHNOLOGY, ELSEVIER, AMSTERDAM, NL, vol. 102, no. 10, 1 March 2011 (2011-03-01), pages 5702 - 5708, XP028407823, ISSN: 0960-8524, [retrieved on 20110305], DOI: 10.1016/ J.BIORTECH.2011.03.001**
• **MAGRÍ ALBERT ET AL: "Feasibility and interest of the anammox process as treatment alternative for anaerobic digester supernatants in manure processing - An over", JOURNAL OF ENVIRONMENTAL MANAGEMENT, vol. 131, 15 December 2013 (2013-12-15), pages 170 - 184, XP028780597, ISSN: 0301-4797, DOI: 10.1016/ J.JENVMAN.2013.09.021**
• **LIU YUAN ET AL: "Upgrading of the symbiosis of Nitrosomanas and anammox bacteria in a novel single-stage partial nitritation-anammox system: Nitrogen removal potential and Microbial characterization", BIORESOURCE TECHNOLOGY, vol. 244, 29 July 2017 (2017-07-29), pages 463 - 472, XP085199096, ISSN: 0960-8524, DOI: 10.1016/ J.BIORTECH.2017.07.156**
• **GÜVEN D ET AL: "IMPLEMENTATION OF THE ANAMMOX PROCESS FOR IMPROVED NITROGEN REMOVAL", JOURNAL OF ENVIRONMENTAL SCIENCE AND HEALTH, PART A: ENV.SCIENCE AND ENGINEER, NEW YORK, NY, US, vol. A39, no. 7, 1 July 2004 (2004-07-01), pages 1729 - 1738, XP008056511**
• **SAMIK BAGCHI ET AL: "Start-up and stabilization of an Anammox process from a non-acclimatized sludge in CSTR", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 37, no. 9, 12 June 2010 (2010-06-12), pages 943 - 952, XP019809571, ISSN: 1476-5535**

Remarks:

The complete document including Reference Table(s) and the Sequence Listing(s) can be downloaded from the EPO website

**Description**

FIELD OF THE INVENTION

The present invention relates to a method for cultivation of bacteria mediating anaerobic ammonium oxidation.

BACKGROUND OF THE INVENTION

Anaerobic ammonium oxidation (hereinafter also abbreviated "anammox") is a microbially catalyzed process of great importance both in the natural nitrogen cycle and in technical applications for removal of fixed nitrogen. In this process bacteria gain energy by oxidizing ammonia, channeling the electrons through an energy-fixing electron transport chain and reducing nitrite to nitrogen gas. The overall reaction is driven by a redox potential difference of about 400 mV (under standard conditions). In comparison with standard nitrogen removal, anammox plants can save energy and co-substrates. To enable anammox, both ammonia and nitrite must be present at the same time. Such conditions occur in natural redox gradient, but in technical plants delicate control is necessary.

In spite of strong efforts, it has not yet been possible to obtain a pure bacterial anammox culture but detailed studies in mixed cultures revealed the phylogenetic affiliation (van Niftrik et al., Anaerobic ammonium-oxidizing bacteria: Unique microorganisms with exceptional properties. Microbiol. Mol. Biol. Rev. 2012, 76 (3), 585-596), the morphology (van Teeseling et al., The S-layer protein of the anammox bacterium Kuenenia stuttgartiensis is heavily o-glycosylated. Front. Microbiol. 2016, 7, 1721), physiology (Oshiki, M. et al., Ecology and physiology of anaerobic ammonium oxidizing bacteria. Environ. Microbiol. 2016, 18 (9), 2784-2796), and biochemistry (Kartal, B.; Keltjens, J. T. Anammox biochemistry: A tale of heme c proteins. Trends Biochem.Sci. 2016, 41 (12), 998-1011; de Almeida et al., B. Membrane-bound electron transport systems of an anammox bacterium: A complexome analysis. Biochimica et Biophysica Acta (BBA) - Bioenergetics 2016, 1857 (10), 1694-704) of the catalyzing bacteria.

One of the first described and best characterized anammox organisms is "*Candidatus* Kuenenia stuttgartiensis", which is a physiological generalist, also able to use iron and manganese as electron acceptors and a wide variety of electron donors for respiration (Strous, M. et al., Deciphering the evolution and metabolism of an anammox bacterium from a community genome. Nature 2006, 440 (7085), 790-794). Growth rates are described to be 0.06-0.23 (doubling times of 3-11 days) (van der Star, W. R. L. et al., The membrane bioreactor: A novel tool to grow anammox bacteria as free cells. Biotechnol. Bioeng. 2008, 101 (2), 286-294; Kartal, B. et al., Chapter 3 - anammox-growth physiology, cell biology, and metabolism. In Adv. Microb. Physiol., Robert, K. P., Ed. Academic Press: 2012; Vol. Volume 60, pp 211-262).

To cultivate anammox microorganisms, different reactor designs have been described. Due to the long doubling times, most research groups cultivated anammox bacteria in biomass-retaining reactors such as upflow anaerobic sludge blanket, or membrane bioreactors to achieve high cell densities and high volumetric anammox reactivity (van der Star et al., The membrane bioreactor: A novel tool to grow anammox bacteria as free cells. Biotechnol. Bioeng. 2008, 101 (2), 286-294; Okamoto, H. et al., Development of a fixed-bed anammox reactor with high treatment potential. Biodegradation 2013, 24 (1), 99-110; Trigo, C. et. al, Start-up of the anammox process in a membrane bioreactor. J. Biotechnol. 2006, 126 (4), 475-487) However, the formation of sludge or granules can strongly influence physiological and biochemical characteristics and only in membrane bioreactors it was possible to grow anammox cells in their planktonic form. However, membrane reactors have drawbacks in complex and expensive design because they require a sludge pump, a gas purging system, a water level sensor, a membrane unit or an effluent pump.

Zhigang Li et al: "Factors affecting the treatment of reject water by the anammox process", Bioresource Technology, Elsevier, Amsterdam, NL, vol. 102, no. 10, 1.03.2011, pages 5702-5708, discloses a method for cultivation of bacteria mediating anammox in a stirred tank anammox reactor, including batch tests, optimization of the nitrite to ammonium ratio, inclusion of mineral salts and determination of the bacteria in the reactor. ZHIGANG uses anammox pellets and a biomass carrier to provide a good environment for microbial growth. Moreover, ZHIGANG introduces air into the reactor. Cells are retained in the reactor by the biomass carrier.

Magrí Albert et al: "Feasibility and interest of the anammox process as treatment alternative for anaerobic digester supernatants in manure processing - An overview", Journal of Environmental Management, vol. 131 , pages 170-184, discloses a method for cultivation of bacteria mediating anammox in a sequencing batch reactor. MAGRí relates to suspended biomass reactors, granular biomass, immobilization of microbial cells, including self immobilization to the surface of a carrier forming biofilm, entrapment and gel pellets.

Liu Yuan et al: "Upgrading of the symbiosis of Nitrosomanas and anammox bacteria in a novel single-stage partial nitritation-anammox system: Nitrogen removal potential and Microbial characterization", Bioresource Technology, vol. 244, 29.07.2017 (2017-07-29), pages 463-472, discloses a method for cultivation of bacteria mediating anammox in a continuous stirred tank reactor, optimization of the nitrite to ammonium ratio, inclusion of mineral salts and determination of the bacteria in the reactor. YUAN grows anammox bacteria on a carrier and introduces air.

Güven D et al: "Implementation of the Anammox Process for Improved Nitrogen Removal", Journal of Environmental Science and Health, part A: Env.Science and Engineer, New York, NY, US, vol. A39, no. 7, 1.07.200$, pages 1729-1738, discloses a method for cultivation of bacteria mediating anammox in a continuous stirred tank reactor, ammonium to nitrite ratio, mineral salts and determination of the bacteria.

Samik Bagchi et al: "Start-up and stabilization of an Anammox process from a non-acclimatized sludge in CSTR", Journal of Industrial Microbiology & Biotechnology ; Official Journal of the Society for Industrial Microbiology, Springer, Berlin, DE, vol. 37, no. 9, 12.06.2010, pages 943-952, discloses a method for cultivation of bacteria mediating anammox in a continuous stirred tank reactor, ammonium to nitrite ratio, mineral salts and determination of the bacteria. Microgranules are used.

OBJECTIVE OF THE INVENTION

The objective of the present invention is to provide an improved cultivation process for anammox microorganisms.

SUMMARY OF THE INVENTION

A general finding of the present invention is that anammox bacteria can be cultivated as planktonic cells in a continuous or semi-continuous stirred-tank reactor (semi-CSTR) with high growth rate ($\mu$), for example of 0.33 d$^{-1}$ at 30°C. By the invention, redox potential inside the reactor can be stabilized, for example at around 10 mV ($\pm$15 mV) (vs standard hydrogen electrode) without gas purging. Reactor headspace pressure can be used as a sensitive and real-time indicator for nitrogen evolution and anammox activity.

Specifically, the present invention provides a method for cultivation of bacteria mediating anaerobic ammonium oxidation, as laid down in independent claim 1. Specific embodiments are illustrated in the dependent claims, description and examples of this invention.

The present invention provides a method for cultivation of bacteria mediating anaerobic ammonium oxidation, the method comprising:

-    performing cultivation of bacteria mediating anaerobic ammonium oxidation (hereinafter also called anammox bacteria) in a stirred tank reactor,

the method further comprising:

    A) feeding nitrite ions and ammonium ions in at least one liquid feed to the stirred tank reactor,
    B) discharging liquid product from the stirred tank reactor, the product comprising bacteria mediating anaerobic ammonium oxidation,

    wherein

    i) feeding in A) and discharging in B) are done continuously, or
    ii) feeding in A) is done repeatedly and discontinuously, and during discharging in B) a fraction of the liquid content from the stirred tank reactor is discharged, which is done repeatedly and discontinuously

    wherein

-    the method comprises degassing the liquid feed(s), and
-    in the cultivation, the bacteria are cultivated as planktonic cells.

Operation mode i) is also called continuous operation.

Operation mode ii) is also called semi-continuous operation. In semi continuous operation feeding in A) (or time periods of feeding) and discharging in C) (or time periods of discharging) are interrupted by operation (or time periods of operation) of the reactor without feeding and without discharging.

A fraction means a part of the liquid content of the reactor. The fraction is also liquid. The fraction has the same composition as an actual liquid content of the reactor.

In another aspect, the present invention provides a liquid fraction or liquid composition obtainable or obtained by the process of the invention.

A liquid composition that can be identical to a liquid fraction taken from the reactor in the method of the invention, or that can be gained from several (e.g. two or more) combined liquid fractions taken from the reactor, can be characterized by comprising a mixture of microorganisms, the mixture comprising bacteria mediating anaerobic ammonium oxidation, wherein the bacteria mediating anaerobic ammonium oxidation are predominantly or completely in planktonic form and the number fraction of bacteria mediating anaerobic ammonium oxidation is at least 50%, preferably at least 60%, more preferably at least 70%, and most preferably at least 80% by number of all microorganisms. The number fraction is preferably measured by Fluorescence *in situ* hybridization.

The present invention discloses the use of said liquid composition for

- cultivating bacteria mediating anaerobic ammonium oxidation,
- treating ammonium-containing waste water, and/or
- stabilizing other microbial population(s) and/or stabilizing the activity of other microbial population(s)

Further disclosed is a plant for cultivation of bacteria mediating anaerobic ammonium oxidation, the plant comprising

- a stirred tank reactor comprising a closure so that the reactor is closed in a gas-tight or substantially gas-tight manner,
- one feed reservoir connected to the stirred tank reactor with a feeding line and containing nitrite ions and ammonium ions in liquid medium, or two feed reservoirs, each of them connected to the stirred tank reactor with a feeding line, wherein one of the two reservoirs contains nitrite ions in a liquid medium and the other of the two reservoirs contains ammonium ions in a liquid medium,
- one discharging line for discharging a liquid fraction from the reactor,
- one gas outlet which could be opened or closed by a gas valve for discharging gaseous product from a headspace of the reactor,
- a pressure sensor for measuring pressure in the headspace,

as well as the use of such plant for cultivation and/or characterization of anammox bacteria, or treatment of ammonium containing waste water.

Treatment of waste water can be done in the plant, particularly in the reactor, so that the reactor is used itself for treatment of waste water that goes into the reactor. Then, the liquid reactor content is waste water which is used in the method of the invention or treated according to a method of the invention. In another embodiment, bacteria from the reactor can be used for treatment of waste water that is somewhere else, particularly in a reservoir, e.g. a large container. Discharged liquid from the reactor, which is a product of the method of the invention and comprising bacteria can be added to waste water.

By the present invention, in its general or specific embodiments, one or more of following advantages or technical effects can be achieved:

- Establish active anammox-bacteria in a simple continuous stirred-tank reactor.
- Homogeneity, sub-cultivation reproducibility, long-term reactor stability and/or high growth rates can be obtained, particularly by eliminating biomass retention
- The system is suitable to characterize physiological properties of the inhabiting microorganisms and to optimize cultivation conditions.
- Reactor effluent or fraction can be used as a stable and homogeneous source for batch cultivations, enrichments and eventually isolation of anammox organisms.
- The stirred-tank reactor can be tightly controlled especially in regard to redox-conditions but also for pH, temperature, nutrients, electron donors and electron acceptors.
- Headspace pressure can be used as a key parameter for reactor control.
- The reactor can produce bacteria in planktonic form at high growth rates. Batch cultivations with the effluent confirmed the success of the approach.

These and further technical effects are illustrated and shown in more detail in the following detailed description and/or in the examples.

DETAILED DESCRIPTION OF THE INVENTION

Bacteria mediating anaerobic ammonium oxidation that can be used in the method of the present invention are in one embodiment selected from the bacterial genera Ca. Kuenenia, Ca. Brocadia, Ca. Jettenia, Ca. Anammoxoglobus, and/or Ca. Scalindua

Anaerobic ammonium oxidation (hereinafter also abbreviated "anammox"), means the following reaction:

$$NH_4^+ + NO_2^- \rightarrow N_2 + 2\,H_2O$$

The present invention can also be called a method for anaerobic ammonium oxidation.

The stirred tank reactor preferably comprises the bacteria in liquid environment, preferably liquid culture medium. The method comprises feeding nitrite ions and ammonium ions in at least one liquid feed to a stirred tank reactor which comprises bacteria mediating anaerobic ammonium oxidation. Anammox-bacteria present in the reactor can be given into the reactor in an earlier step of the method, which is also called "inoculation".

During the cultivation, anaerobic ammonium oxidation reaction and proliferation/production of bacteria takes place.

When discharging a liquid fraction, a part of the content of the stirred tank reactor (the content also called "liquid phase" or "liquid volume" in the reactor) is discharged. In a specific embodiment, one fraction is ≤10 vol.%, preferably ≤ vol.5%, more preferably ≤ vol.3% of the liquid volume in the reactor. A lower range of one fraction that could be combined with any of the before mentioned upper ranges is, 0.01 vol.%, or 0.1 vol.%. So, the fraction is for example 0.01 - 10 vol.%, 0.01 - 5 vol.%, 0.01 - 3 vol.%, 0.1 - 10 vol.%, 0.1 - 5 vol.%, or 0.1 - 3 vol.%. The volume of discharged fraction may be constant, from cycle to cycle, or vary. A constant or essentially constant volume is preferred. The content of the stirred tank reactor, or liquid volume in the reactor, means in this connection the liquid volume during batch operation, when no feed is added and no content is discharged. If the content of the stirred tank reactor is varied, then the above percentage relates to the actual content before discharging.

Mentioned percentage is also called "exchange ratio". The exchange ratio may be constant over several cycles, or non-constant. The exchange ratio may decrease, for example from cycle to cycle or over several cycles. Change of the exchange ratio may be done by changing the volume flow of feed and/or discharge, and/or by changing the first period of time.

The volume of the feed can be different or the same as the volume of discharged fraction, preferably the same, either in continuous operation or in semi-continuous operation. If the content of the stirred tank reactor is varied in semi-continuous operation, then the above percentage relates to the actual content before feeding.

Continuous operation of the stirred tank reactor means that the reactor is operated as a continuous stirred tank reactor (CSTR).

Semi-continuous operation means that feeding and discharging are done discontinuously. Feeding is interrupted, once or several times, and discharging is interrupted.

Semi-continuous operation particularly means in the present invention that

- during a first period of time feeding and discharging is done, either sequentially in any order, or simultaneously or in part simultaneously.
- during a second period of time no feeding and no discharging is done. This means that the reactor is operated in batch mode.

These steps may be repeated once or more, preferably several times.

During feeding, the feed may be added in one stream or in several streams comprising different ingredients, which is explained in more detail below.

The term "In part simultaneously" means that feeding and discharging have an overlap in time, wherein the time of feeding and discharging are not identical.

In a specific embodiment, semi-continuously operation of the stirred tank reactor is done in following manner:

a) in a first period of time, liquid feed is added and liquid fraction comprising bacteria is discharged, which is done either simultaneously or in part simultaneously,
or sequentially in any order,
b) for a second period of time, the stirred tank reactor is operated in a batch mode. during this period of time no feed is added and no content from the reactor is discharged.

The sequence of steps a) and b) may be repeated several times.

The designation "first" in the first period of time designates a period of time during which feeding and discharging is done (simultaneously, in part simultaneously, or sequentially in any order) but does not designate at length of such period. The length of a first period may stay constant or vary in comparison of several "first periods".

The designation "second" in the second period of time designates period of time during which batch operation is done, but does not designate at length of such period. The length of a second period may stay constant or vary in comparison of several "second periods".

The first and/or second period of time may change in their length when the sequence of steps a) and b) is repeated. Steps a) and b), taken together, are also called a "cycle". From cycle to cycle, the first and second periods of time may change in their length, so that consequently the total time of the cycle may change. Particularly, the second period of time may change in its length. In a specific embodiment, the second period of time is decreased, for example from cycle to cycle or over several cycles.

In an embodiment of the method of the invention, the hydraulic retention time in the reactor is at least 1 day, preferably at least 2 days, even more preferably at least 3 days. Upper limits of a retention time, which can be combined in all possible combinations with each of these lower limits are 100 days, 50 days, 14 days or 10 days. Hydraulic retention time (HRT) is calculated as follows:

$$HRT = \text{liquid volume in reactor/volume flow rate}$$

In continuous operation (operation mode i) as indicated above), volume flow rate is a continuous flow rate.

In semi-continuous operation (operation mode ii) as indicated above), volume flow rate for calculation of HRT means the following:

$$\text{volume flow rate} = \text{volume of discharged fraction/cycle time.}$$

The cycle time is the sum of above indicated first period of time and second period of time (time periods in in above steps a) and b)).

In one embodiment, the reactor is closed in a gas-tight manner and the method comprises:

- measuring a pressure change in a headspace of the reactor in order to monitor activity of the bacteria.

In the anammox reaction, nitrogen is produced. By the produced nitrogen, pressure in the headspace of the reactor is increased. Thus, activity of the anammox-bacteria and progress of anammox-reaction can be monitored by measuring the pressure change in the headspace of the gas tight reactor.

In one embodiment, the method comprises

- continuously or discontinuously releasing nitrogen-containing gas from the headspace, the nitrogen formed in anaerobic ammonium oxidation reaction.

This embodiment, which can be particularly combined with previously mentioned embodiment, has the benefit, that nitrogen can be discharged when the pressure in the headspace reaches or exceeds a given level. The gaseous atmosphere the headspace predominantly comprises nitrogen that is formed in anammox-reaction, but may also comprise other gases, like carbon dioxide and others.

Releasing nitrogen-containing gas makes possible to control pressure and that gaseous atmosphere in the headspace is less disturbed.

As mentioned before, feed may be added in one stream or in several streams comprising different ingredients. In one embodiment of the invention, feeding is done in following manner:

- feeding a first liquid feed comprising nitrite ions
- feeding a second liquid feed comprising ammonium ions.

A further embodiment comprises:

- feeding a third liquid feed comprising ions of alkaline metal and/or earth alkaline metal.

A benefit that can be reached by these embodiments is that precipitation and abiotic reaction of different components with each other outside of the reactor can be decreased, minimized or even avoided. The liquid feed, or liquid feeds, may comprise further ingredients, such as counter ions, ions of different metals (in summary also called trace elements), vitamins and the like.

The basic liquid of the liquid compositions mentioned herein is preferably water.

The method comprises degassing the liquid feed(s), particularly degassing mentioned first and second liquid feeds, and, if applicable (i.e. if used), mentioned third liquid feed.

Degassing results in beneficially constant redox potential inside the reactor.

A benefit of the process of the invention is a product that comprises anammox bacteria in predominantly, or almost exclusively, planktonic form. Planktonic form means that the bacteria occur isolated in liquid environment, not in clusters.

A liquid composition can be obtained or be obtainable from the process of the invention. As mentioned before, the liquid composition can be a liquid fraction obtained in the process, or a combination of such fractions.

Mentioned liquid composition or fraction of the process of the invention, or combined fractions, can beneficially be used for a variety of purposes, such as

i) Cultivating anammox-bacteria, mediating anaerobic ammonium oxidation. Here, the liquid composition or fraction may be used as a starting culture. The effluent of the reactor may be used for the start-up of commercial anammox-reactors
ii) Treating ammonium-containing waste water, and/or
iii) Stabilizing other microbial population(s) and/or stabilizing the activity of other microbial population(s).

The above-mentioned uses lead to following additions to a method of the invention:

In one embodiment, the method the invention further comprises

- feeding discharged liquid product from continuous operation or at least one of the liquid fraction from the stirred tank reactor to a further reactor and
- cultivating bacteria mediating anaerobic ammonium oxidation in the further reactor.

In one embodiment, the method the invention further comprises

- isolating bacteria mediating anaerobic ammonium oxidation from discharged liquid product. The discharged liquid product may be liquid product from continuous operation or liquid product from discontinous operation, particularly at least one of a discharged liquid fraction from the stirred tank reactor.

In this embodiment, the method of the invention is beneficial for gaining Anammox bacteria in isolated form for any purpose, for example for further examination, analysis or research. Isolation techniques from liquid phase are known to the skilled person, for example centrifugation.

In one embodiment, the method the invention further comprises

- treating ammonium-containing waste water with discharged liquid product. The discharged liquid product may be liquid product from continuous operation or liquid product from discontinous operation, particularly at least one of a discharged liquid fraction from the stirred tank reactor.

In one embodiment, the method of the invention further comprises

- feeding discharged liquid to a bioreactor containing microbial population(s),

The discharged liquid product may be liquid product from continuous operation or liquid product from discontinous operation, particularly at least one of a discharged liquid fraction from the stirred tank reactor. Feeding discharged liquid to a bioreactor containing microbial population(s) leads to stabilization of microbial population(s) in the bioreactor. So, the method comprises, as an effect of the feeding step: stabilizing the microbial population(s) and/or the activity of said microbial population(s) in said bioreactor.

Stabilizing microbial populations may mean particularly the following: Microbial populations in another reactor may be a anammox bacteria population, for example a small or still not stable population. Discharged liquid product from the method of the invention may be added to stabilize, promote, boost or even establish (significant) anammox activity.

In aforementioned embodiments, the term "at least one of" expresses that one or more fractions can be used in further process steps, wherein the fractions can be combined before further use or not.

Discharged liquid product from continuous operation may be used directly or it may be collected and/or stored. Discharged liquid product means liquid content from the reactor that is discharged from the reactor in continuous operation. Discharged liquid product comprises anammox bacteria. Products of anammox reaction which are gaseous, like nitrogen, are not encompassed by the term "liquid product".

Microbial populations may comprise anammox bacteria and/or other microorganisms. This embodiment is useful for the continuous supply of anammox organisms to commercial bioreactors, of arbitrary type, to stabilize their microbial populations and activity over longer periods of time.

Temperatures for carrying out methods of the invention are temperatures wherein anammox bacteria are active and can survive. Exemplary temperatures are 15-40°C, preferably 25-35°C.

Further aspects and embodiments concerning the plant for cultivation are as follows:

The term "gas tight" or "substantially gas-tight" comprises that case, or means, that some materials for a closure may allow

**EP 3 502 234 B1**

a migration of gas in trace amounts and/or over a long period of time, for example weeks or months. However, a closure allowing only such minor, and usually negligible, migration of gas, in also undertood to be gas-tight or at least substantially gas tight.

The stirred tank reactor may comprise bacteria mediating anaerobic ammonium oxidation in liquid medium.

A third feed reservoir connected to the stirred tank reactor may be present, containing ions of alkaline metal and/or earth alkaline metal in liquid medium.

The plant may comprise a controller, or controlling unit, designed or intended for receiving data from the pressure sensor and controlling opening/closing of the gas valve.

The plant may comprise a reservoir for discharged liquid fraction(s) comprising bacteria from the reactor, which is also called effluent.

Further embodiments and aspects of the invention are also disclosed the following examples and figures, which are not to be construed as limiting for the present invention. The content of examples, even if specifically presented, may be generalized to create further embodiments of the invention.

BRIEF DESCRIPTION OF THE FIGURES

Fig. 1:     Schematic layout of a semi-continuous stirred-tank reactor;

Fig. 2:     Anammox activity in a sludge- and granule-free planktonic batch cultures after three transfers. Shown are means $\pm$ SD of triplicate cultures. Nitrogen evolution and cell growth were not measured here;

Fig. 3:     Fluorescence *in situ* hybridization of the reactor culture with FAM-labeled Eub338-mix, cy5-labeled AMX820 and counter stained with DAPI;

Fig. 4:     Influence of different effectors on anammox activity of reactor effluent. (A) Salt (NaCl); (B) Nitrite (NaNO$_2$); (C) Sulfide (Na$_2$S); (D) Phosphate (mixture of K$_2$HPO$_4$ and KH$_2$PO$_4$, pH 7.2); (E) Chloramphenicol; (F) Methylene blue. Gas production rates were normalized against positive controls with no effectors and 3 mM nitrite which produced 70-120 mL N$_2$ L$^{-1}$ d$^{-1}$ at 30°C.

Fig. 5:     Hydraulic retention time in the reactor during start-up;

Fig. 6:     Gas pressure and redox changes over time in the reactor after discontinuing medium amendments;

Fig. 7:     Epifluorescence microscopic image of the SYBR Green-stained reactor culture;

Fig. 8:     Influencing parameters on cell counting by epifluorescence microscopy;

Fig. 9     gas production in cultures with reactor effluent amended with different chloramphenicol concentrations.

BRIEF DESCRIPTION OF SEQUENCES

SEQ ID NO: 1     Forward primer 338F
SEQ ID NO: 2     Forward primer 338F-AMX
SEQ ID NO: 3     Reverse primer 1392R
SEQ ID NO: 4     Reverse primer 806R
SEQ ID NO: 5:     Forward primer 341F
SEQ ID NO: 6:     Reverse primer 785R

EXAMPLES

1. Material and methods

1.1 Bacterial inoculum and media

Anammox sludge granules from a sequencing batch reactor treating landfill leachate were handled strictly anoxic. Both, initial batch cultivation and reactor cultivation were done in an anoxic defined synthetic mineral medium containing vitamins but no reducing agents. For initial batch cultivation in serum bottles, cultures were inoculated with 10% vol/vol. To inoculate from granules, bottles were opened inside an anaerobic tent and the inoculum was transferred with a sterile spatula. The reactor was semi-continuously fed from three different solutions with ammonium, nitrite and mineral salts as the principal components. All feeding solutions were prepared strictly anoxic by degassing and bubbling with nitrogen.

1.2 Reactor components and operation

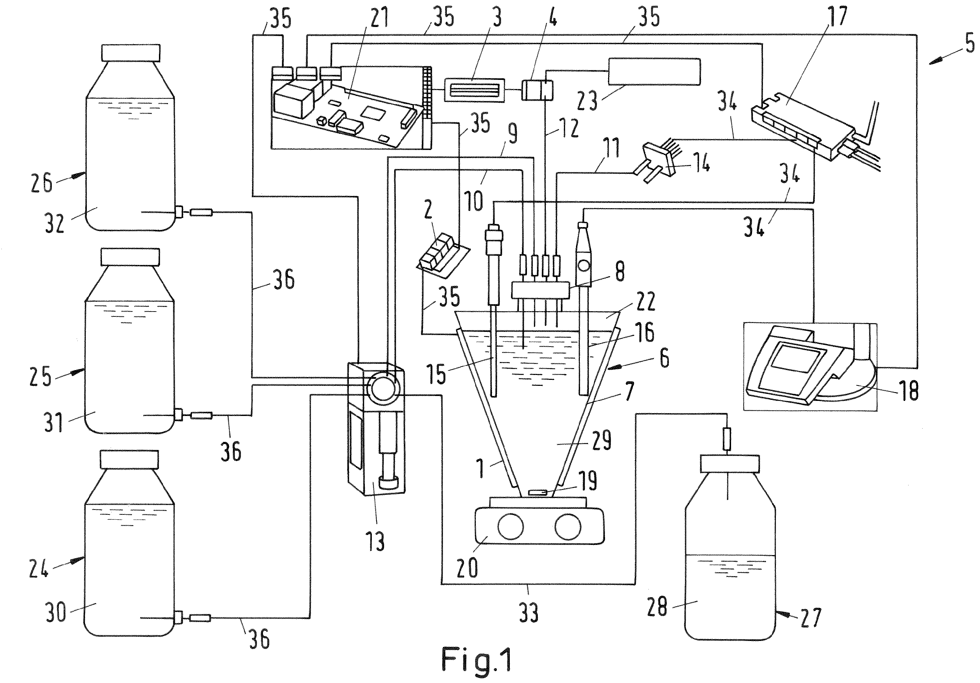

The plant 5 comprised the stirred tank reactor 6. The vessel 7 was an inverted 2.4 L Erlenmeyer flask with ports for medium amendments, sampling and gas pressure measurements which were all done automatically and controlled via a Raspberry Pi microprocessor (Fig. 1 1).

The reactor vessel 7 had GL45 and two GL27 screw ports on the top (Fig. 1). The GL45 port was closed with closure 8 (2 cm thick butyl rubber septum) through which two liquid lines 9 (feed line), 10 (discharge line)and two gas lines 11, 12 were connected via 23G 1.5-inch Luer Lock needles. The two liquid lines 9, 10 were connected with PEEK tubing (green, 0.75 mm inner diameter) to a PSD/4 syringe pump 13 (Hamilton, Bonaduz, Switzerland) equipped with a 5 mL syringe (Model 1010.5 TLL with UHMWPE plunger) and a six-port distribution valve (Hamilton, Bonaduz, Switzerland) to inject or extract liquids, respectively, to or from the reactor. The two-line design was chosen to avoid dead volume in the liquid lines. The two gas lines 11, 12 were connected to a pressure sensor 14 (MPX5100DP, Conrad, Germany) and a two-way electric gas valve 4 (Model 225T011, NResearch, USA) for gas release, respectively. The two GL27 ports were used to connect an InLab Redox Micro redox electrode 15 (Mettler Toledo, USA) and an InLab Expert Pro pH/temperature sensor 16 (Mettler Toledo, USA), respectively. The voltage signal from the redox electrode 15 was collected using a LabJack T7Pro analog/digital converter 17 (LabJack, USA) with 22-bit resolution once every 3 seconds. Readings from the pH/temperature sensor 16 were monitored using a SevenMulti S40 pH meter 18 (Mettler Toledo, Switzerland) once every 7 seconds. The redox electrode 15 used 3 M KCl and saturated AgCl as reference electrolyte, giving a reference electrode potential of 205 mV (vs standard hydrogen electrode) at 30°C. Redox potential calibration was done with a 475 mV redox standard buffer (Hamilton, Bonaduz, Switzerland). The liquid in the reactor was constantly stirred using a magnetic stirrer 19 at 120 rpm, and was kept at constant temperature (30±0.1°C) with a heating blanket 1. All electric signals were transferred to a Raspberry Pi 3 (model B, Raspberry Pi Foundation, UK) microcomputer 21 with microSD card storage for monitoring and data storage (controller). Control of the pumping, heating, and gas release was done on the basis of the measurements and controlled by the Raspberry Pi controller 21. The controlling program script was written in Python 2.

Anammox activity was followed indirectly by monitoring the pressure with the electronic differential pressure sensor 14 which translates the pressure difference into an analogous voltage signal between 0 and 5 V (MPX5100DP, Conrad, Germany). Detectable pressure range of the sensor 14 is between 0 to 100 kPa. Voltage output at atmospheric pressure (when both ports of the sensor are connected to atmosphere), minimum voltage output, and voltage change per kPa are intrinsic values associated with each pressure sensor (153 mV, 69 mV and 45 mV kPa$^{-1}$ for the sensor installed on the reactor). For a reactor headspace 22 of 400 mL, 1 mV voltage change corresponds to 87.7 $\mu$L of gas production (22.2 Pa pressure change). The voltage signal was converted with the LabJack converter 17 with 22 bit resolution, and was collected once every 3 seconds. The noise level (standard deviation) of the signal after data smoothing over a period of 2 min was ~0.05 mV, giving the detection limit of 0.15 mV, corresponding to 3.3 Pa (13 $\mu$L of gas production). In order to precisely quantify nitrogen evolution for calculation of reactor nitrogen mass balance, the gas releasing valve 4 was connected to a gas collection bottle 23 working on the principle of water displacement by adsorbing the $CO_2$ share of the gas with 50 mM NaOH.

All medium bottles (first/second/third feed reservoirs 24, 25, 26) and the effluent bottle (reservoir 27 for discharged liquid composition 28) were pressurized with a gas mixture of 80% $N_2$ and 20% $CO_2$ at 10 kPa.

The reactor contained 2 L culture liquid 29 and 400 mL headspace 22. Periodically every 43-100 minutes, depending on the chosen hydraulic retention time, 20 mL reactor liquid 29 was pumped from the reactor 6 into the effluent bottle 27, and was replaced with 20 mL fresh medium (10 mL 40 mM nitrite medium/ first liquid feed 30; 5 mL 120 mM ammonium medium/ second liquid feed 31; and 5 mL salt medium/ third liquid feed 32).

All cultivations were done in synthetic mineral media without undefined additions. Batch cultivation medium contained per liter 1 g NaCl, 0.5 g $MgCl_2 \cdot 6H_2O$, 0.2 g $KH_2PO_4$, 0.835 g $NH_4Cl$, 0.3 g KCl, 15 mg $CaCl_2 \cdot 2H_2O$, 14.2 mg $Na_2SO_4$, 2.292 g TES (N-[Tris(hydroxymethyl)methyl]-2-aminoethanesulfonic acid), 1 mL trace element solution, and 1 mL Se/W solution. This medium was boiled for 20 min under nitrogen gas, cooled to room temperature and anoxically amended with 2.52 g L$^{-1}$ $NaHCO_3$. The medium was then distributed to serum bottles, capped with butyl rubber stoppers and autoclaved. After autoclaving, 3 mL of 1 M $NaNO_2$, and 2 mL vitamin solution were added to each liter of autoclaved medium from filter-sterilized solution.

The reactor was fed with three different solutions to avoid precipitation and abiotic reaction of the different components with each other outside of the reactor (Fig. 1): i) the "salt medium" (= third liquid feed 32) containing per liter 0.4 g MgCl$_2$.6H$_2$O, 0.8 g KH$_2$PO$_4$, 1.2 g KCl, and 60 mg CaCl$_2$·2H$_2$O; ii) the "ammonium medium" (=second liquid feed 31) containing per liter 6.42 g NH$_4$Cl (120 mM), 8 mL trace element solution, and an additional 4 mL 0.02 g L$^{-1}$ CuCl$_2$·2H$_2$O; and iii) "nitrite medium" (=first liquid feed 30) containing per liter 2.26 g NaNO$_2$ (40 mM), 1.18 g NaHCO$_3$ (20 mM), 28.4 mg Na$_2$SO$_4$, 4 mL Se/W solution, and 8 mL vitamin solution. The components were prepared under non-sterile conditions and were purged with under nitrogen gas using a fritted glass sparger for at least 20 min at room temperature. NaNO$_2$, NH$_4$Cl, and NaHCO$_3$ were added in powder form inside an anaerobic tent. The bicarbonate concentration in the feeding solution was 10 mM final concentration.

One liter of trace element solution contained 10 mL 25% HCl, 1.5 g FeCl$_2$·4H$_2$O, 0.19 g CoCl$_2$·6H$_2$O, 0.1 g MnCl$_2$·4H$_2$O, 70 mg ZnCl$_2$, 6 mg H$_3$BO$_3$, 36 mg Na$_2$MoO$_4$·2H$_2$O, 24 mg NiCl$_2$·6H$_2$O, and 2 mg CuCl$_2$·2H$_2$O. One liter of Se/W solution contained 0.5 g NaOH, 6 mg Na$_2$SeO$_5$·5H$_2$O, and 8 mg Na$_2$WO$_4$·2H$_2$O. One liter of vitamin solution contained 10 mg biotin, 10 mg folic acid, 100 mg pyridoxine hydrochloride, 25 mg (-)-riboflavin, 25 mg thiamine hydrochloride, 25 mg nicotinic acid, 25 mg D-pantothenic acid hemicalcium salt, 25 mg p-aminobenzoic acid, 25 mg thioctic acid, and 25 mg vitamin B$_{12}$.

Ammonium was always in excess to maintain a low redox potential and non-toxic nitrite concentrations. The buildup of headspace pressure was continuously monitored and when it reached 7.71 kPa (sensor voltage output of 500 mV), gas valve 4 opened for 30 s to release gas into the gas collection bottle 23. This low threshold was set to minimize disturbance of the CO$_2$ layer in the reactor headspace 22.

1.3 Incubations of reactor effluent with inhibitory compounds

60 mL nitrogen-flushed glass bottles were anoxically filled with 50 mL reactor effluent and closed with butyl rubber septa and aluminum crimp caps. Effectors were anoxically added with sterile syringes through the septa or directly added to the bottles as powder. Except for the experiment in which nitrite was varied, 3 mM nitrite was added at time 0. Each bottle was directly connected to a MPX5100DP pressure sensor via a 0.41 mm Luer Lock needle. All sensors were electrically connected to a LabJack converter for data reading and storage. Pressure sensors were read every 3 seconds. In total, twelve such bottles were set up for each experiment, allowing the simultaneous testing of five different effector concentrations in duplicate plus two bottles as negative controls without effector.

Experiments were incubated for 24 hours after which 3 mL air was injected into each bottle for calibration of the pressure sensor readings. Gas production rates were calculated as mL per day across 1,000 consecutive data points (3,000 seconds). Maximum gas production was typically reached after 10-14 hours. The same time frame was chosen for all twelve bottles in one experiment to calculate anammox activities from the slopes.

1.4 Analytical and microscopic techniques

Ammonium concentrations were determined with a modified salicylate method (Kandeler, E.; Gerber, H. Short-term assay of soil urease activity using colorimetric determination of ammonium. Biol. Fertil. Soils 1988, 6 (1), 68-72). In brief, samples were diluted to a ammonium concentration below 0.15 mM, and 200 $\mu$L of these diluted samples were sequentially mixed in a 96-well microplate with 50 $\mu$L bleach solution (120 mM NaOH, 0.15% w/v sodium hypochlorite) and 50 $\mu$L catalyst solution (120 mM NaOH, 10% w/v sodium salicylate, 0.04% w/v sodium nitroferricyanide). A$_{650}$ was measured on a Synergy 2 microplate reader (Biotek, USA). Nitrite concentrations were determined with the Griess reagent kit (Molecular Probes) according to the manufacturer's instructions. Nitrate concentrations were determined on a Dionex DS-120 ion chromatography with an IonPac AS4A-SC column and an eluent containing 0.7 mM NaCO$_3$ and 0.7 mM NaHCO$_3$ at a flow rate of 1 mL min$^{-1}$.

To measure volatile suspended solids (VSS), 200 mL reactor effluent was filtered through 47-mm diameter Whatman GF/F glass fiber filter (pore size 0.7 $\mu$m, GE Healthcare, USA) previously baked at 550°C for 1.5 h. After filtration the filter was dried overnight at 105°C and weighed to obtain the total suspended solid (TSS) value. Then the filter was combusted at 550°C for 1.5 h to obtain the VSS value from the difference of the weight. VSS analysis was done in triplicate.

Direct epifluorescence microscopic counting of SYBR-green stained cells on agarose-coated slides and fluorescence *in situ* hybridization (FISH) of cells with 16S-rRNA-targeting probes are described hereinafter:

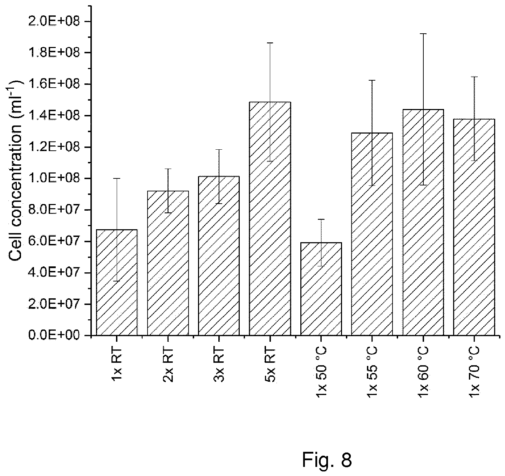

Direct microbial cell counting was done from images taken with a digital camera mounted on an epifluorescence microscope. SYBR Green I DNA stain in DMSO was purchased from ThermoFisher (USA), and diluted 100$\times$ in TE buffer (10 mM Tris, 1 mM EDTA, pH 7.2). 6.5 $\mu$L of this 100$\times$ diluted stock solution was mixed with 20 $\mu$L culture liquid, before 18 $\mu$L was loaded onto agarose-coated slides as described earlier (Marco-Urrea, Eet al., Transformation and carbon isotope fractionation of tetra- and trichloroethene to trans-dichloroethene by Dehalococcoides sp. strain CBDB1. Environ. Sci. Technol. 2011, 45, 1555-1562; Adrian, L et al. Growth of Dehalococcoides strains with chlorophenols as electron acceptors. Environ. Sci. Technol. 2007, 41 (7), 2318-2323). Images were taken after SYBR Green stained cells were evenly distributed on the slides. Automation of the counting was done with ImageJ and VBA macros as described in the above mentioned publications by Adrian and Marco-Urrea. The higher concentration of SYBR Green stain compared to previous studies was chosen because a higher fraction of anammox cells were stained under this concentration. A similar result was achieved by an increase of the incubation temperature (Fig. 8) but this option was not chosen due to potential cell lysis effects.

For fluorescence *in situ* hybridization (FISH) 0.8 mL fresh reactor effluent was fixed with 0.2 mL 20% paraformaldehyde (final paraformaldehyde concentration 40 g $L^{-1}$) for three hours at 4°C, mixed with 1 mL absolute ethanol, and stored at -20°C. The mixture was then filtered onto a 25-mm diameter 0.2-$\mu$m pore GTTP polycarbonate filter (Millipore, Eschborn, Germany). Hybridization was done with the FAM-labeled probe set Eub338-mix (containing Eub338, Eub338-II, and Eub338-III, 40% formamide, final concentration 5 ng $\mu L^{-1}$ each) for Eubacteria and the cy5-labeled probe AMX820 (40% formamide, final concentration 3 ng $\mu L^{-1}$) for anammox bacteria (Daims, H. et al., The domain-specific probe Eub338 is insufficient for the detection of all bacteria: Development and evaluation of a more comprehensive probe set. Syst. Appl. Microbiol. 1999, 22 (3), 434-444; Schmid, M. et al., Molecular evidence for genus level diversity of bacteria capable of catalyzing anaerobic ammonium oxidation. Syst. Appl. Microbiol. 2000, 23 (1), 93-106.). Probes were from Eurofins (Ebersberg, Germany). We used 4',6'-diamidino-2-phenylindole (DAPI) dilactate (1.2 $\mu$g mL$^{-1}$) for counter-staining. FISH images were taken with a Zeiss Imager Z2 AXIO fluorescence microscope at 1,000 $\times$ magnification.

1.5 Microbial community analyses

One milliliter of culture liquid was centrifuged at 10,000 g for 15 min at 4°C, and the resulting pellet was used for extraction of genomic DNA using DNeasy Blood and Tissue kit (QIAGEN, Hilden, Germany) according to manufacturer's instructions, except that 20 $\mu$L of lysozyme (100 mg mL$^{-1}$) were added to facilitate cell lysis. Extracted DNA samples were stored at -20°C and used as template for clone library construction and Illumina sequencing.

PCR amplification of 16S rRNA genes for clone library construction was done for 30 cycles using the forward primer (338F 5'-ACT CCT ACG GGA GGC AGC AG-3' (SEQ ID NO: 1) and 338F-AMX 5'-ACT CCT ACG GGA GGC TGC AG-3' (SEQ ID NO: 2), 0.05 $\mu$M each) and the reverse primer 1392R 5'-ACG GGC GGT GTG TRC-3' (SEQ ID NO: 3) (0.1 $\mu$M) with the annealing temperature of 55°C. Purified PCR products were ligated onto pGEM-T vector (Promega) which was later transformed into *E. coli* competent cells. Clones were sequenced by Sanger sequencing using the primers provided with the vector.

For Illumina amplicon sequencing of the DNA from the initial batch culture, V3-V4 region of 16S rRNA genes were amplified using the forward primer 338F 5'-ACT CCT ACG GGA GGC AGC AG-3' (SEQ ID NO: 1) and the reverse primer 806R 5'-GGA CTA CHV GGG TWT CTA AT-3' (SEQ ID NO: 4). Illumina MiSeq sequencing (300 bp paired-end reads) was performed by BGI (Shenzhen, China). For Illumina amplicon sequencing of the DNA from the reactor effluent, V3-V4 region of the 16S rRNA gene were amplified using the forward primer 341F 5'-CCT ACG GGN GGC WGC AG-3' (SEQ ID NO: 5) and the reverse primer 785R 5'-GAC TAC HVG GGT ATC TAA TCC-3' (SEQ ID NO: 6) (Klindworth, A.; Pruesse, E.; Schweer, T.; Peplies, J.; Quast, C.; Horn, M.; Glöckner, F. O. Evaluation of general 16S ribosomal RNA gene PCR primers for classical and next-generation sequencing-based diversity studies. Nucleic Acids Res. 2013, 41 (1), e1). Illumina MiSeq sequencing (300 bp paired-end reads) was performed by LGC Genomics (Berlin, Germany). All sequence reads were processed using the NGS analysis pipeline of the SILVA rRNA gene database project (SILVAngs 1.3) (Quast, C.; Pruesse, E.; Yilmaz, P.; Gerken, J.; Schweer, T.; Yarza, P.; Peplies, J.; Glöckner, F. O. The SILVA ribosomal RNA gene database project: improved data processing and web-based tools. Nucleic Acids Res. 2013, 41 (Database issue), D590-D596). OTU clustering threshold was 0.98 sequence identity. The classification was performed by a local nucleotide BLAST search against the non-redundant version of the SILVA SSU Ref dataset (release 128). Classification threshold was 0.93 sequence similarity. Visualization of microbial population abundance was done with Krona (Ondov, B. D.; Bergman, N. H.; Phillippy, A. M. Interactive metagenomic visualization in a Web browser. BMC Bioinformatics 2011, 12 (1), 385).

2. Results

2.1 Batch cultivation and microbial community analysis

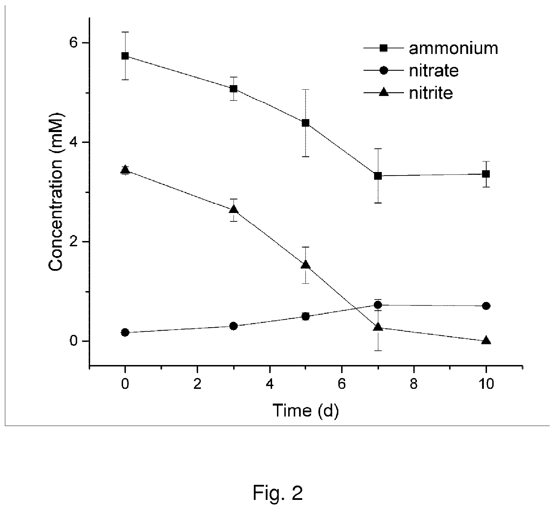

Suspended granules (30 mL) were obtained from an anammox reactor and stored anoxically at 4°C for almost one year. Then, 5 mL of the granules were transferred to 90 mL of batch cultivation medium containing 3 mM nitrite and 4.6 mM ammonium. After two months of incubation, nitrite was depleted in the bottle, and 10% v/v inocula were transferred to fresh medium. After another two such transfers an active anammox culture free from granules and sludge was obtained. The culture catalyzed conversion of 3 mM nitrite in ten days with concurrent decrease of ammonium concentrations (Fig. 2). A population analysis by sequencing clones from a 16S rRNA gene clone library indicated that about a quarter of the population (26.7%) was affiliated with "Ca. Kuenenia stuttgartiensis", whereas 56.7% were affiliated with the genus *Pseudomonas,* as shown in the following table:

*Population composition of the initial anammox culture after three transfers in batch cultures analyzed by clone library sequencing*

*The DNA sample was collected on day 3 of incubation.*

| Population | Number of Clones | Abundance |
|---|---|---|
| *"Candidatus* Kuenenia stuttgartiensis" | 8 | 26.7% |
| *Pseudomonas bauzanensis* | 14 | 46.7% |
| *Pseudomonas xiamenensis* | 3 | 10% |
| *Others* | 5 | 16.7% |

With the same DNA sample, an Illumina amplicon sequencing was performed, confirming that the culture was dominated by organisms described to catalyze the anammox process ("Ca. Kuenenia stuttgartiensis", 17.2%) and *Pseudomonas* (70.3%), while the other OTUs were all at lower population shares than 2%.

To obtain sufficient culture liquid to inoculate the reactor, 100 mL of granule-free culture liquid was anoxically transferred to 900 mL of medium containing 3 mM nitrite. After 15 days of incubation the nitrite concentration was below the detection limit and the culture was used for reactor inoculation.

2.2 Reactor cultivation of "Ca. Kuenenia stuttgartiensis"

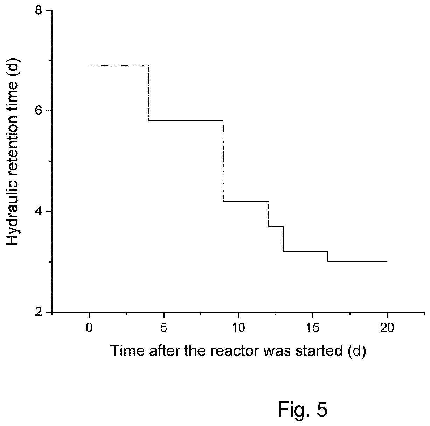

A gas-tight semi-continuous stirred tank reactor (semi-CSTR) was constructed allowing strictly anoxic reactor operation and real-time monitoring of pressure, redox potential, pH and temperature (Fig. 1, cf. methods). It allowed the amendment of nitrite and ammonium as the two main energy-providing molecules from separate medium bottles. To start the reactor, the 1-L stem culture described above was anoxically mixed with 1 L fresh batch cultivation medium containing 3 mM nitrite and transferred to the reactor. The reactor was started with a hydraulic retention time of 6.9 day, and an influent nitrite and ammonium concentration of 20 mM and 30 mM, respectively. During operation, the concentration of nitrite in the reactor effluent was constantly close to zero and continuously gas was produced demonstrating that the injected nitrogen compounds were used in the reactor. When the hydraulic retention time was decreased by stepwise increasing the frequency of the 1% medium replacement (exchanging 1% fractions, 20 mL) procedure the effluent nitrite concentration stayed below 0.05 mM (Fig. 5). In this way, the hydraulic retention time was shortened to 3 days after 16 days of operation.

The chosen medium composition and reactor operation effected that the pH was constantly between 7.3 and 7.6.

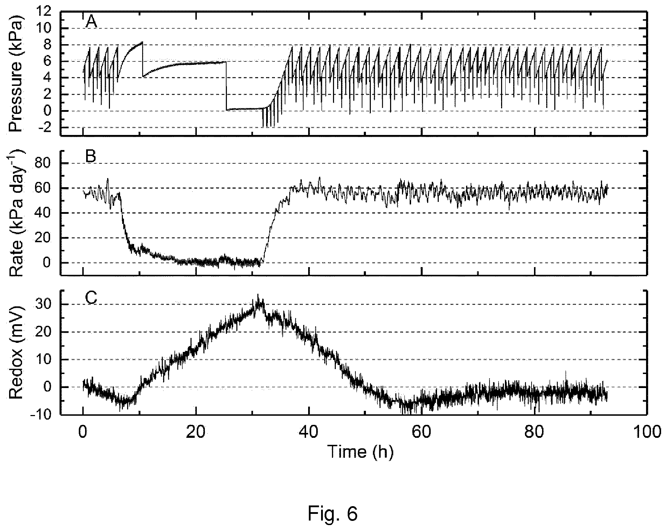

Initial experiments demonstrated that anammox activity strongly depended on the redox potential of the medium (data not shown) and it was therefore planned to also provide a reducing agent to control the redox potential. However, long-time operation of the reactor for over one year demonstrated that with the chosen reactor design, degassing of feeding solutions and under anoxic operation no redox adjustment was necessary as the redox potential inside the reactor stayed at around 10 mV $\pm$ 15 mV vs the standard hydrogen electrode (SHE). When the reactor flow was halted, the redox potential rose gradually to ~30 mV within 26 h (Fig. 6). Fig. 6 shows gas pressure and redox changes over time in the reactor after discontinuing medium amendments. In this experiment the pump, normally working at a hydraulic retention time of 3 days, was stopped in the time period between 6 and 32 hours. During this time pressure development decreased towards zero while the redox potential increased constantly to a value of 30 mV. Both parameters recovered to their original values after the pumping resumed within 30 hours. (A) Headspace pressure of the reactor. When the overpressure reached 7.71 kPa the gas was released automatically via an electric valve, (B) Gas production rate, in kPa day-1 calculated from a window of 480 consecutive pressure readings, and (C) Redox potential (vs SHE) of the reactor liquid; data were smoothened over 20 consecutive readings. The temperature was between 29.9 and 30.1°C, pH between 7.4 and 7.6 throughout the test. Once the reactor flow resumed, the redox potential instantly stopped rising and slowly returned to the original level. After interrupting the reactor flow it took about 10 h for gas production to completely cease, and another 5 h to regain full gas production rate after resuming the reactor flow.

2.3 Reactor recovery

The reactor effluent was collected in anoxic 2-liter bottles and stored in the dark at 4°C to be used later for inhibitor tests (see below) or as inoculum when the reactor collapsed. During one-year operation under experimental conditions, the reactor intentionally collapsed three times under three different critical conditions: 1) after the reactor was incubated for 24 h at 38.6°C without feeding nitrite, 2) after a pulse of 0.2 mM $Na_2S$ was added, and 3) after a pulse of 65 $\mu$M $FeCl_2$ was

added. The first two conditions led to complete loss of the anammox activity, while the third condition resulted in the formation of a thick precipitate in the reactor which was presumably ferric oxides and consecutively stopped reactor operation. After such reactor collapses, the reactor could be reproducibly regenerated with the stored eluent bottles. The activity in the effluent bottles was recovered, as judged from pressure production rates, within only 2 days when a bottle was warmed up to room temperature, spiked with 3 mM nitrite and incubated at 30°C. Effluent could be stored at 4°C for at least three months without startup delay.

2.4 Microbial community composition during reactor operation

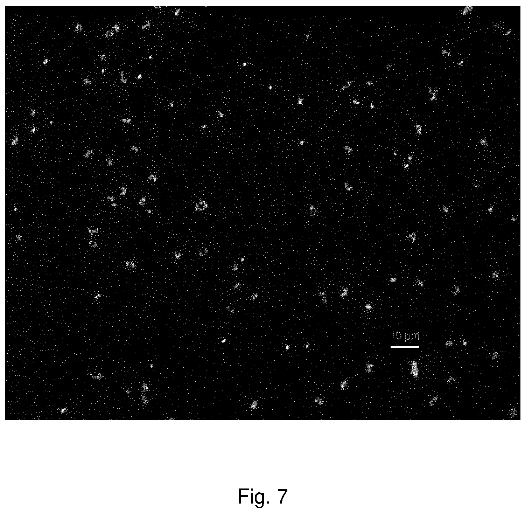

Under steady state conditions with a hydraulic retention time of 3 days, no granules developed in the reactor and the liquid changed to an orange color as described for other anammox cultures. The microbial cell density within the reactor was at $7.6 \pm 1.3 \times 10^7$ mL$^{-1}$. Direct microscopic analysis of SYBR Green stained cells also showed that the cells were not assembling in cell aggregates indicating that all cells in the reactor were in a planktonic form (Fig. 7). The majority of the cells showed the typical morphology of anammox cells with a ring or "c"-type shape indicating the presence of a DNA-free anammoxosome. The fluorescence intensity was influenced by the concentration of SYBR Green dye applied and the incubation temperature (Fig. 8). Fig. 8 shows influencing parameters on cell counting by epifluorescence microscopy. Shown is the effect of the amount of SYBR Green I DNA stain and the temperature at which cells were incubated with SYBR Green on cell counts. RT: room temperature. $1\times$: 1.3 μL $100\times$ diluted SYBR Green stock solution for 20 μL culture liquid. $5\times$: 6.5 μL SYBR Green for 20 μL culture liquid. All measured samples were taken from the same batch of anammox reactor effluent, and all conditions were done in triplicates. For each replicate, at least 15 images were taken for cell counting.

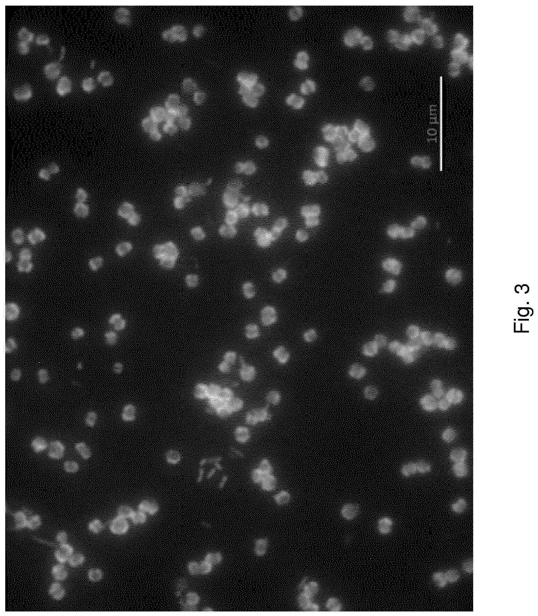

To quantify the abundance of anammox-catalyzing organisms in the established reactor culture, FISH was conducted using FAM-labeled EUB338-mix and cy5-labeled AMX820 probes for Eubacteria and anammox bacteria, respectively, and DAPI as DNA-counterstain. Samples taken from the reactor running steadily at ~0.33 d$^{-1}$ dilution rate (hydraulic retention time of 3 days) showed that the abundance of anammox organisms increased to ~85% (Fig. 3). In Fig. 3 Fluorescence *in situ* hybridization of the reactor culture with FAM-labeled Eub338-mix (green, color not shown in this Fig.), cy5-labeled AMX820 (red, color not shown in this Fig.) and counter stained with DAPI (blue, color not shown in this Fig.). Anammox cells are light blue (color not shown in this Fig.) on the edges due to DAPI-stained high DNA content in the cytoplasm ('riboplasm' in Planctomyetes); the center of the anammox organisms ('anammoxosome') is DNA-free and stains orange/brown (color not shown in this Fig.) due to three overlapping weak signals from the two FISH probes and DAPI from the over/underlaying riboplasm. Cells that do not hybridize with AMX820 are blue or green (color not shown in this Fig.).

A second Illumina amplicon sequencing of the established reactor culture showed that the relative share of anammox organisms had increased to around 75%, while bacteria of the genus *Pseudomonas* almost disappeared (0.007%) and were replaced by heterotrophs such as *Methyloversatilis* (10.6%), *Azoarcus* (2.8%), *Melioribacter* (3.4%), *Rubellimicrobium* (2.0%), and several genera within the *Lentimicrobiaceae* (1.5%). If the genome of the "*Ca.* Kuenenia stuttgartiensis" strain in the reactor contains only one 16S rRNA gene copy as described for the draft genome assembly of "Ca. Kuenenia stuttgartiensis" (Strous, M. et al., Deciphering the evolution and metabolism of an anammox bacterium from a community genome. Nature 2006, 440 (7085), 790-794.) and the other detected strains in the reactor culture have the 16S rRNA gene copy numbers as given in *rrnDB* (Lee, Z. M. P.; Bussema, C.; Schmidt, T. M. rrnDB: documenting the number of rRNA and tRNA genes in bacteria and archaea. Nucleic Acids Res. 2009, 37 (Database issue), D489-D493), the adjusted anammox cell fraction in the reactor was around 87%.

2.5 Stoichiometry of anaerobic ammonium oxidation in the reactor

To calculate the stoichiometry of the anammox process, a correlation between the cell density and VSS was obtained first. On day 30, the reactor effluent had a cell density of $8.5 \times 10^7$ mL$^{-1}$ and a VSS value of 48.1 ($\pm 1.2$) mg L$^{-1}$, giving a per cell weight of $5.65 \times 10^{-13}$ g VSS which was used to convert cell density to VSS in the nitrogen mass balance experiments below.

During a two-week monitoring period, all nitrogen-containing species flowing into and out of the reactor were measured (cf. below table) including ammonium and nitrite in the reactor influent, ammonium, nitrite, nitrate and biomass in the reactor effluent, and nitrogen gas evolution. The total cell density was converted to anammox cell density using the anammox abundance observed above (87%). The elemental composition of the biomass was assumed as $CH_2O_{0.5}N_{0.15}$ according to published results for "*Ca.* Brocadia" (Strous, M.; Heijnen, J. J.; Kuenen, J. G.; Jetten, M. S. M. The sequencing batch reactor as a powerful tool for the study of slowly growing anaerobic ammonium-oxidizing microorganisms. Appl. Microbiol. Biotechnol. 1998, 50 (5), 589-596). During the monitoring period, 252.5 and 249.7 mmole of nitrogen flowed in and out of the reactor, respectively, representing an almost closed mass balance.

*Mass balance of nitrogen in the reactor monitored over two weeks*

| | Incoming | | Outgoing | |
|---|---|---|---|---|
| **Species** | **N (mmole)** | **Species** | | **N (mmole)** |
| Nitrite | 101.0±1.0 | Nitrogen gas | | 160.2±2.0 |
| Ammonium | 151.5±2.0 | Nitrate | | 18.8±1.0 |
| | | Ammonium | | 69.7±2.5 |
| | | Biomass-N* | | 0.98±0.10 |
| **Total-In** | **252.5** | **Total-Out** | **249.7** | |

* This was calculated based on the biomass formula $CH_2O_{0.5}N_{0.15}$ given for "Ca. Brocadia" (Strous, M. et al., The sequencing batch reactor as a powerful tool for the study of slowly growing anaerobic ammonium-oxidizing microorganisms. Appl. Microbiol. Biotechnol. 1998, 50 (5), 589-596)

Data reconciliation of the above measurements balancing nitrogen in- and output (van der Heijden, R.; Heijnen, J.; Hellinga, C.; Romein, B.; Luyben, K. Linear constraint relations in biochemical reaction systems: I. Classification of the calculability and the balanceability of conversion rates. Biotechnol. Bioeng. 1994, 43 (1), 3-10) resulted in a corrected anammox stoichiometry as shown in Equation 1. Based on this equation, biomass yield was 1.94 g VSS per mole of consumed ammonium.

$$1\,NH_4^+ + 1.263\,NO_2^- + 0.081\,HCO_3^- + 0.105\,H^+$$
$$= 1.006\,N_2 + 0.239\,NO_3^- + 0.081\,CH_2O_{0.5}N_{0.15} + 2.012\,H_2O$$

Equation 1

2.6 Incubations of the reactor effluent with various effectors

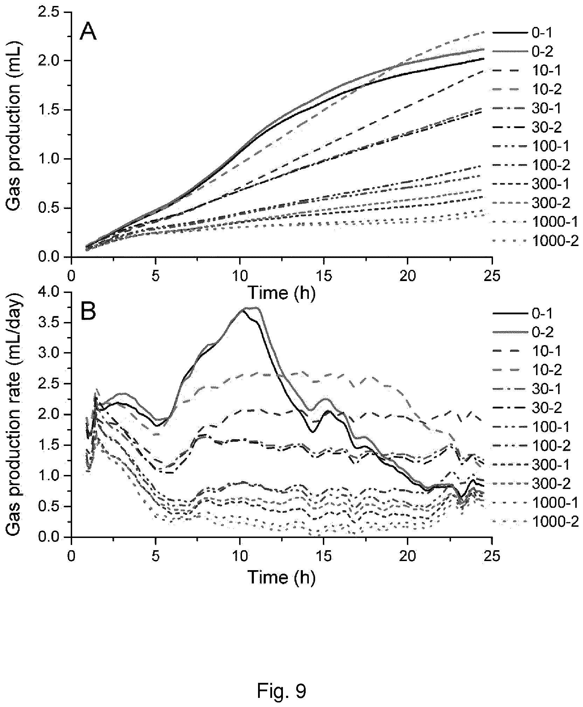

An important advantage of the CSTR design was the homogeneity of the reactor effluent which can be used for physiological characterization of anammox bacteria. For that the reactor effluent was incubated in closed bottles and monitored the pressure build-up in the headspace over time. Consistency of the obtained data between replicate bottles and correlation of pressure build-up and effector concentration was demonstrated in an experiment where tested the effect of chloramphenicol on anammox activity was tested (Fig. 9). Fig. 9 shows the gas production in cultures with reactor effluent amended with different chloramphenicol concentrations. Legend: the number before the dash indicates the chloramphenicol concentration in mg $L^{-1}$, the number behind the dash indicates the number of the replicate. (A) Gas production; (B) Gas production rate calculated in a window of 1,000 consecutive data points (equivalent to 3,000 seconds). In cultures without chloramphenicol maximum gas production rates were reached after 10.4 h and this time point was chosen to compare with the slopes in the chloramphenicol-amended cultures.

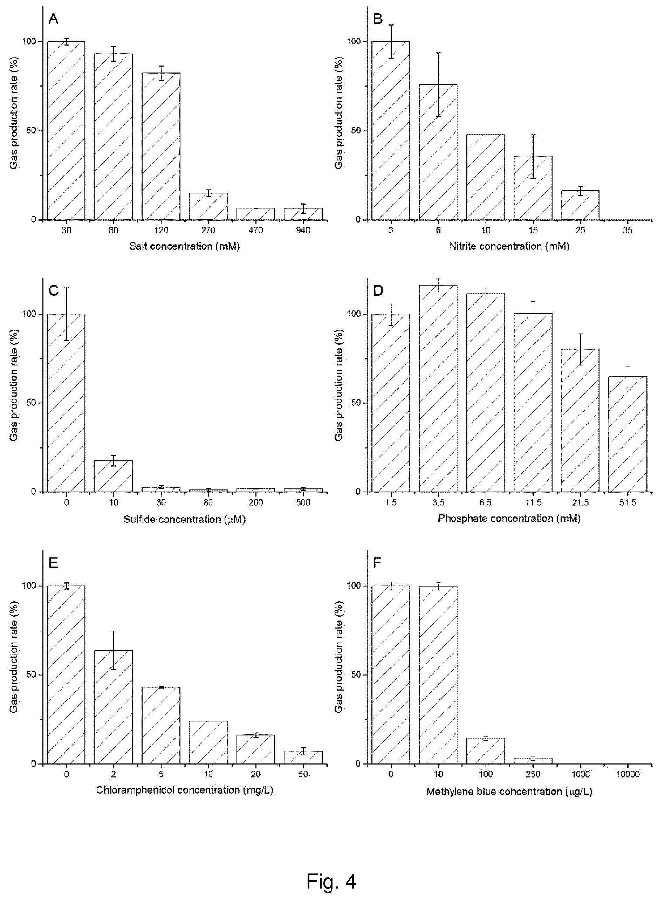

In total, six effectors were tested in this study: salt (NaCl, 30-940 mM), nitrite (3-35 mM), sulfide (Na$_2$S, 0-500 μM), phosphate (mixture of K$_2$HPO$_4$ and KH$_2$PO$_4$, pH 7.2, 1.5-51.5 mM), chloramphenicol (0-1 g $L^{-1}$), and methylene blue (0-10 mg $L^{-1}$) (Fig. 4). In brief, IC50 values were 183 mM (NaCl), 9.6 mM (nitrite), 5 μM (sulfide), >51.5 mM (phosphate), 19 mg $L^{-1}$ (chloramphenicol), and 53 μg $L^{-1}$ (methylene blue). No apparent inhibition was observed with salt concentrations up to 120 mM, while 270 mM caused 75% inhibition. Nitrite at 6 mM caused 24% inhibition. Sulfide severely inhibited anammox activity already at the very low concentration of 5 μM. Chloramphenicol induced 57% inhibition at a concentration of 30 mg $L^{-1}$. An increase of anammox activity by 16% was observed when the phosphate concentration was increased from 1.5 mM to 3.5 mM. At 6.5 mM phosphate, anammox activity was 11% higher than in the controls at 1.5 mM. Therefore, a higher phosphate concentration may enhance anammox activity also in the reactor. Still 65% of the anammox activity was preserved at the highest tested concentration of 51.5 mM phosphate. Whereas methylene blue, a redox indicator potentially useful for anammox cultivation, caused no apparent inhibition of anammox activity at 10 μg $L^{-1}$, 100 μg $L^{-1}$ methylene blue caused 86% inhibition.

3. Discussion

3.1 Semi-CSTR is a promising reactor design for anammox cultivation

In this study, a semi-CSTR was successfully used for the cultivation of anammox cells in their planktonic form. This reactor design produces planktonic anammox cells. Membrane bioreactors have been used to cultivate "Ca. Kuenenia stuttgartiensis" ($\mu$=0.23 d$^{-1}$), *"Ca.* Brocadia sapporoensis" ($\mu$=0.33 d$^{-1}$), "Ca. Brocadia sinica", and "Ca. Scalindua sp." (van der Star et al., The membrane bioreactor: A novel tool to grow anammox bacteria as free cells. Biotechnol. Bioeng. 2008, 101 (2), 286-294; Oshiki, M. et al., Cultivation of planktonic anaerobic ammonium oxidation (anammox) bacteria using membrane bioreactor. Microbes Environ. 2013, 28 (4), 436-443; Lotti, T. et al., Faster through training: The anammox case. Water Res. 2015, 81, 261-268). Cultivation in semi-CSTR of the invention let to growth of "Ca. Kuenenia stuttgartiensis" at a slightly higher growth rate of $\mu$=0.33 d$^{-1}$ than the previously reported highest growth rate for this species. Compared to membrane bioreactors, the semi-CSTR design is much simpler and does not require a sludge pump, a gas purging system, a water level sensor, a membrane unit or an effluent pump (because medium and effluent pumps were combined in the system in one syringe pump). Especially omission of the membrane unit is advantageous, as the membrane contributes significantly to the investment costs in membrane bioreactors and also to the maintenance costs as the membrane unit needs to be periodically cleaned after clogging or formation of biofilms on the membrane fibers.

The nitrogen loading rate in the semi-CSTR (in kg-N m$^{-3}$ d$^{-1}$) increases when the hydraulic retention time is decreased. However, at fixed nitrogen concentration in the influent, the nitrogen loading rate was limited in the semi-CSTR because the hydraulic retention time cannot be set below the inverse of the growth rate ($1/\mu_{max}$) of the anammox organisms without diluting them out. Membrane bioreactors as well as other biomass-retaining reactors decouple the solid retention time from the hydraulic retention time, and therefore hydraulic retention time could be further reduced. Full scale anammox plants can achieve nitrogen removal rates of more than 0.26 kg-N m$^{-3}$ d$^{-1}$ for the anammox stage (Mao, N. et al., Engineering application of anaerobic ammonium oxidation process in wastewater treatment. World J. Microbiol. Biotechnol. 2017, 33 (8), 153.). For a CSTR to achieve a nitrogen removal rate of 0.26 kg-N m$^{-3}$ d$^{-1}$ with a hydraulic retention time of 3 days, at least 780 mg L$^{-1}$ (55.7 mM) nitrogen should be removed from the influent, corresponding to an influent nitrite concentration of 24.6 mM (55.7/(1+1.263), Equation 1). The semi-CSTR of the invention already had a very similar influent nitrite concentration of 20 mM. Therefore, the CSTR design might even be useful in full scale wastewater treatment where the influent contains a high amount of nitrogen or where a simple reactor design is preferred. Even if the full-scale application for wastewater treatment cannot be realized, the CSTR design is very useful to precisely study the microbiology, physiology and biochemistry of anammox bacteria due to its simple structure and planktonic cell yield. Moreover, without the need for a gas supply system in the semi-CSTR of the invention, it is possible to monitor headspace pressure change for rapid determination of anammox activity inside the reactor (Fig. 6).

3.2 Influence of oxygen and the redox potential on anammox

Although anammox organisms can survive phases of oxic treatment in the lab, anammox activity is extremely sensitive to oxygen. At the same time, anammox organisms are dependent on the presence of nitrite which is not available under strictly anoxic conditions. Therefore, anammox bacteria are typical redox gradient organisms both in freshwater and marine habitats where they thrive in so-called "oceanic oxygen minimum zones". In a membrane bioreactor system, continuous bubbling with 95% argon and 5% $CO_2$ was needed to maintain an oxygen-free environment (Kartal, B. et al., Chapter four - cultivation, detection, and ecophysiology of anaerobic ammonium-oxidizing bacteria. In Methods Enzymol., Martin, G. K., Ed. Academic Press: 2011; Vol. Volume 486, pp 89-108; Lotti, T. et al., Physiological and kinetic characterization of a suspended cell anammox culture. Water Res. 2014, 60, 1-14). In contrast to this intensive and expensive gas sparging, the CSTR design of the invention using degassed feeding solutions did not require continuous gas purging to obtain stable growth of anammox cells.

In previous experiments oxygen also had a striking effect on the growth form of Planktomycetes: Cells of "Ca. Brocadia sapporoensis" started to aggregate in a membrane bioreactor when the oxygen loading rate was > 0.68 $\mu$mole-$O_2$ L$^{-1}$ d$^{-1}$ (Lotti, T et al., Physiological and kinetic characterization of a suspended cell anammox culture. Water Res. 2014, 60, 1-14. In the semi-CSTR of the invention, cells of "Ca. Kuenenia stuttgartiensis" were in their planktonic form throughout all reactor operations (Fig. 3 and Fig. 7). Even after exposure to air (21% oxygen) for 2 hours, no aggregates were formed in the reactor effluent as observed by epifluorescence microscopy (images not shown). In summary, "Ca. Brocadia sapporoensis" seems to aggregate more easily upon oxygen exposure than "Ca. Kuenenia stuttgartiensis".

Apart from dissolved oxygen, the redox potential is a parameter that greatly influences anammox activities. Previous studies showed that anammox activities were maximal around 0 mV (vs SHE) (Lackner, S. et al., 'Swinging ORP' as operation strategy for stable reject water treatment by nitritation-anammox in sequencing batch reactors. Chem. Eng. J. 2012, 180, 190-196.; Yang, J. et al., Oxidation-reduction potential (ORP) as a control parameter in a single-stage partial nitritation/anammox process treating reject water. J. Chem. Technol. Biotechnol. 2015, 91 (10), 2582-2589). Indeed, the redox potential within the semi-CSTR stabilized at around 10 mV (vs SHE) as long as the reactor was in operation (Fig. 6).

The self-stabilization of the redox potential without external manipulation such as reductant addition or gas purging is an interesting phenomenon, and the underlying mechanism is worth further investigation.

3.3 Growth rate and growth yield

Since the demonstration of relatively fast growth of anammox bacteria in membrane bioreactors anammox organisms have no longer been considered as slow-growing microorganisms. So far, the highest reported maximum growth rates for anammox bacteria were 0.33 d$^{-1}$ for "Ca. Brocadia" (Zhang, L.; Narita, Y.; Gao, L.; Ali, M.; Oshiki, M.; Okabe, S. Maximum specific growth rate of anammox bacteria revisited. Water Res. 2017, 116 (1), 296-303; Lotti, T et al., Faster through training: The anammox case. Water Res. 2015, 81, 261-268) and 0.18 d$^{-1}$ for "Ca. Jettenia" (Lotti et al.) corresponding to a steady state solid retention time of 3 days and 5.6 days, respectively. With the reactor design of the invention the hydraulic retention time was gradually decreased to 3 days ($\mu$=0.33 d$^{-1}$) within 16 days of reactor operation (Fig. 5). This was achieved without intensive optimization of temperature, pH, or the medium recipe. Adjusting phosphate concentration could enhance anammox activity as shown in the batch test (Fig. 4D).

When the culture exhibited the highest growth rate, the specific anammox activity was 3.05 g NH$_4$ g$^{-1}$ VSS d$^{-1}$. This value is close to the one reported for "Ca. Brocadia sapporoensis" (3.38 g NH$_4$ g$^{-1}$ VSS d$^{-1}$) (Lotti, T. et al., Faster through training: The anammox case. Water Res. 2015, 81, 261-268). Given that these two species exhibited similar maximum growth rates and specific anammox activity, these values may have approached the limit for freshwater anammox cells.

3.4 Anammox stoichiometry

Anammox stoichiometry for "Ca. Kuenenia stuttgartiensis" was determined as given in Equation 1 based on a two-week reactor operation. Compared to two previously determined stoichiometric equations for anammox "Ca. Brocadia sapporoensis" (Equation 2) (Lotti, T. et al., Physiological and kinetic characterization of a suspended cell anammox culture. Water Res. 2014, 60, 1-14.) and "Ca. Brocadia anammoxidans" (Equation 3) (Strous, M. et al., The sequencing batch reactor as a powerful tool for the study of slowly growing anaerobic ammonium-oxidizing microorganisms. Appl. Microbiol. Biotechnol. 1998, 50 (5), 589-596), the consumption ratio of nitrite over ammonium for "Ca. Kuenenia stuttgartiensis" (1.263) was between the two *Brocadia* species, while biomass production for "*Ca.* Kuenenia stuttgartiensis" was slightly higher. The latter led to slightly lower specific anammox activity.

$$1\,\mathrm{NH_4^+} + 1.146\,\mathrm{NO_2^-} + 0.071\,\mathrm{HCO_3^-} + 0.057\,\mathrm{H^+}$$
$$= 0.986\,\mathrm{N_2} + 0.161\,\mathrm{NO_3^-} + 0.071\,\mathrm{CH_{1.74}O_{0.31}N_{0.2}} + 2.002\,\mathrm{H_2O}$$

Equation 2

$$1\,\mathrm{NH_4^+} + 1.32\,\mathrm{NO_2^-} + 0.066\,\mathrm{HCO_3^-} + 0.13\,\mathrm{H^+}$$
$$= 1.02\,\mathrm{N_2} + 0.26\,\mathrm{NO_3^-} + 0.066\,\mathrm{CH_2O_{0.5}N_{0.15}} + 2.03\,\mathrm{H_2O}$$

Equation 3

3.5 Microbial population changes during enrichment

Bacteria of the genus *Pseudomonas,* which accounted for 70.3% of the total population in the batch cultures used as reactor inoculum, almost disappeared after three-months of reactor operation. This relative high abundance of *Pseudomonas* species in the batch cultures might be due to their denitrifying activity making use of the much higher nitrite concentrations in the batch cultures compared to the concentrations in the reactor. *Pseudomonas xiamenensis* was reported to be a denitrifying bacterium. *Pseudomonas bauzanensis* was described as a non-denitrifying bacterium, but its genome (NCBI Accession No. JFHS00000000.1) contains the full set of denitrification genes suggesting that its denitrification activity might have not yet been discovered. The disappearance of potentially denitrifying cells of the genus *Pseudomonas* might therefore be due to the fact that the dilution rate of the reactor exceeded their maximum growth rate under reactor conditions.

A number of heterotrophs emerged during reactor operation, including *Methyloversatilis* which accounted for more than 10% of the total population in the reactor after 3 months. *Methyloversatilis* strains were facultative methylotrophs growing on a variety of organic compounds. *Chloroflexi* were often reported to co-exist with anammox bacteria and scavenge organic compounds derived from anammox bacterial cells. However, in reactors with fast dilution rates and active anammox populations, decayed anammox cells should be minimal. Indeed, in the reactor of the invention, *Chloroflexi* accounted for only 0.019% of the total population.

Anammox abundance in the semi-CSTR in this study reached around 87% as shown by Illumina amplicon sequencing and FISH, lower than what membrane bioreactor systems usually achieved (>95%) (Kartal, B. et al., Chapter four - cultivation, detection, and ecophysiology of anaerobic ammonium-oxidizing bacteria. In Methods Enzymol., Martin, G. K., Ed. Academic Press: 2011; Vol. Volume 486, pp 89-108). This is probably because the semi-CSTR was fed with only 20 mM nitrite while membrane bioreactor influent typically contained 60 mM nitrite. A higher nitrite concentration led to higher anammox biomass yield while the heterotrophs in the reactor were limited by trace amounts of organics in the influent. The abundance of anammox bacteria is expected to increase when influent nitrite is elevated and when the hydraulic retention time is further reduced but this was not the goal in the current study.

3.6 Effluent incubations

The monitored effects in incubations of reactor effluent with inhibitors are due to the combined inhibitory effects on anammox enzymatic activity and on anammox cell growth. However, because the incubation time was relatively short and because only 3 mM nitrite was added (except for tests of nitrite effects) compared to reactor influent containing 20 mM nitrite, cell growth during the experiment should be low.

The present results that salt concentrations of 120-270 mM start to inhibit anammox activity is consistent with previous findings suggesting a salt tolerance range of between 50 and 200 mM (Oshiki, M., et al., Ecology and physiology of anaerobic ammonium oxidizing bacteria. Environ. Microbiol. 2016, 18 (9), 2784-2796). Slightly higher sensitivity for nitrite and sulfide was found in the reactor effluent compared to the literature. While it was found here that nitrite caused severe inhibition at 6 mM, previous reports described "Ca. Kuenenia stuttgartiensis" to be tolerant to 13-25 mM nitrite (Egli, K. et al., Enrichment and characterization of an anammox bacterium from a rotating biological contactor treating ammonium-rich leachate. Arch. Microbiol. 2001, 175(3), 198-207; Dapena-Mora, A. et al.; Evaluation of activity and inhibition effects on anammox process by batch tests based on the nitrogen gas production. Enzyme Microbiol. Technol. 2007, 40 (4), 859-865).

The very strong inhibition with sulfide observed was even stronger than reported with planktonic cells from membrane bioreactors (Russ, L et al., Interactions between anaerobic ammonium and sulfur-oxidizing bacteria in a laboratory scale model system. Environ. Microbiol. 2014, 16 (11), 3487-3498; Carvajal-Arroyo, J. M. et al., Inhibition of anaerobic ammonium oxidizing (anammox) enrichment cultures by substrates, metabolites and common wastewater constituents. Chemosphere 2013, 91 (1), 22-27), and was orders of magnitude lower than values obtained with aggregate cells (Jin, R.-C. et al., The effect of sulfide inhibition on the ANAMMOX process. Water Res. 2013, 47(3), 1459-1469; Brooks, M. H. et al., Inhibition of existing denitrification enzyme activity by chloramphenicol. Appl. Environ. Microbiol. 1992, 58 (5), 1746-1753). Chloramphenicol has a direct inhibitory effect on denitrification enzymes, and is therefore of interest for the study of anammox bacteria which compete for nitrite with denitrifying bacteria. Contrasting results on the effects of chloramphenicol on anammox activity of "Ca. Kuenenia stuttgartiensis " showed that 1 g $L^{-1}$ chloramphenicol caused no apparent inhibition (Dapena-Mora, A, Evaluation of activity and inhibition effects on anammox process by batch tests based on the nitrogen gas production. Enzyme Microbiol. Technol. 2007, 40 (4), 859-865), and that in a sequencing batch reactor 20 mg $L^{-1}$ chloramphenicol caused 25% drop in reactor efficiency and 75% drop in specific anammox activity after 4 days (Fernández, I. et al., Operation of an anammox SBR in the presence of two broad-spectrum antibiotics. Process Biochemistry 2009, 44 (4), 494-498). In the present experiments 30 mg $L^{-1}$ caused 57% inhibition which is more in line with the latter study.

In the present invention, the reactor was dominated by an organism closely related to "*Candidatus* Kuenenia stuttgartiensis" (~87% abundance) as shown by Illumina amplicon sequencing and fluorescence *in situ* hybridization. Epifluorescence microscopy demonstrated that all cells were in their planktonic form. Mass balance analysis revealed a ratio of consumed nitrite to consumed ammonium of 1.263 and a biomass yield of 1.94 g volatile suspended solids per mole of consumed ammonium. Batch experiments with the reactor effluent showed that anammox activities were sensitive to sulfide (IC50 = 5 $\mu$M) and chloramphenicol (IC50 = 19 mg $L^{-1}$). This invention shows that semi-CSTR is a powerful tool to study and produce anammox bacteria.

LIST OR REFERENCE SIGNS:

1     heating blanket
2     relay for heating blanket
3     relay for gas valve

| 4  | gas valve |
| 5  | plant |
| 6  | stirred tank reactor |
| 7  | vessel |
| 8  | closure/ rubber septum |
| 9  | liquid line/feed line |
| 10 | liquid line/discharge line |
| 11 | gas line |
| 12 | gas line/ gas outlet |
| 13 | pump |
| 14 | pressure sensor |
| 15 | redox electrode |
| 16 | pH/temperature sensor |
| 17 | A/D converter |
| 18 | pH meter |
| 19 | stirrer |
| 20 | stirring device |
| 21 | controller |
| 22 | headspace |
| 23 | gas collection bottle |
| 24 | first feed reservoir |
| 25 | second feed reservoir |
| 26 | third feed reservoir |
| 27 | reservoir for discharged liquid composition |
| 28 | liquid composition comprising bacteria |
| 29 | culture liquid/reactor liquid |
| 30 | first liquid feed |
| 31 | second liquid feed |
| 32 | third liquid feed |
| 33 | discharging line |
| 34 | sensor lines |
| 35 | electric lines |
| 36 | liquid lines/feed lines |

**Claims**

1. A method for cultivation of bacteria mediating anaerobic ammonium oxidation, comprising

   - performing cultivation of bacteria mediating anaerobic ammonium oxidation in a stirred tank reactor (6), the method further comprising:

      A) feeding nitrite ions and ammonium ions in at least one liquid feed (30, 31, 32) to the stirred tank reactor (6),
      B) discharging liquid product from the stirred tank reactor (6),

   wherein

      i) feeding in A) and discharging in B) are done continuously, or
      ii) feeding in A) is done repeatedly and discontinuously, and when discharging in B) is done, a fraction of the liquid content in the stirred tank reactor (6) is discharged, which is done repeatedly and discontinuously,

   **characterized in that**:

      - the method comprises degassing the liquid feed(s) (30, 31, 32), and
      - in the cultivation, the bacteria are cultivated as planktonic cells.

2. The method of claim 1, wherein in ii) the fraction of the liquid content is ≤10 vol.%, preferably ≤3 vol% of the liquid content.

3. The method of one or more of the preceding claims, wherein in ii) operation of the stirred tank reactor (6) is done in following manner:

a) in a first period of time, liquid feed (30, 31, 32) is added and liquid fraction is discharged, which is done either simultaneously or in part simultaneously,
or sequentially in any order,
b) for a second period of time, the stirred tank reactor (6) is operated in a batch mode,

wherein the sequence of steps a) and b) is repeated at least one time and wherein the first and/or second period of time may change in their length when the sequence of steps a) and b) is repeated.

4. The method of one or more of the preceding claims, wherein the reactor (6) is closed in a gas-tight manner and the method comprises:

- measuring a pressure change in a headspace (22) of the reactor (6) in order to monitor activity of the bacteria.

5. The method of one or more of the preceding claims, comprising

- continuously or discontinuously releasing nitrogen-containing gas from the headspace (22), the nitrogen formed in anaerobic ammonium oxidation reaction.

6. The method of one or more of the preceding claims, wherein feeding is done in following manner:

- feeding a first liquid feed (30) comprising nitrite ions
- feeding a second liquid feed (31) comprising ammonium ions.

7. The method of one or more of the preceding claims, comprising

- feeding a third or further liquid feed (32) comprising ions of alkaline metal and/or earth alkaline metal.

8. The method of one or more of the preceding claims, further comprising

- feeding discharged liquid product from continuous operation or at least one of the liquid fraction from the stirred tank reactor (6) to a further reactor and
- cultivating bacteria mediating anaerobic ammonium oxidation in the further reactor.

9. The method of one or more of the preceding claims, further comprising

- isolating bacteria mediating anaerobic ammonium oxidation from discharged liquid product.

10. The method of one or more of the preceding claims, further comprising

- treating ammonium-containing waste water with discharged liquid product.

11. The method of one or more of the preceding claims, further comprising

- feeding discharged liquid product to a bioreactor containing microbial population(s).

**Patentansprüche**

1. Verfahren zur Kultivierung von Bakterien mittels anaerober Ammoniumoxidation, umfassend

- Durchführen einer Kultivierung von Bakterien mittels anaerober Ammoniumoxidation in einem Rührkesselreaktor (6),
wobei das Verfahren ferner umfasst:

A) Zufuhr von Nitrit-Ionen und Ammonium-Ionen in Form mindestens einer Flüssigzufuhr (30, 31, 32) zu dem Rührkesselreaktor (6),

B) Entnahme von Flüssigprodukt aus dem Rührkesselreaktor (6),

wobei

i) Zufuhr in A) und Entnahme in B) kontinuierlich erfolgen, oder

ii) Zufuhr in A) wiederholt und diskontinuierlich erfolgt, und wenn eine Entnahme in B) erfolgt, ein Anteil des flüssigen Inhalts in dem Rührkesselreaktor (6) entnommen wird, und dies wiederholt und diskontinuierlich erfolgt,

**dadurch gekennzeichnet, dass**:

- das Verfahren ein Entgasen der Flüssigkeitszufuhr(en) (30, 31, 32) umfasst, und
- in der Kultur die Bakterien als planktonische Zellen kultiviert werden.

2.  Verfahren nach Anspruch 1, wobei in ii) der Anteil des flüssigen Inhalts ≤10 vol%, bevorzugt ≤3 vol% des flüssigen Inhalts ist.

3.  Verfahren nach einem oder mehreren der vorstehenden Ansprüche, wobei in ii) ein Betrieb des Rührkesselreaktors (6) wie folgt erfolgt:

a) In einem ersten Zeitraum wird Flüssigzufuhr (30, 31, 32) hinzugegeben und ein Flüssiganteil entnommen, was entweder gleichzeitig oder teilweise gleichzeitig oder in Abfolge in einer beliebigen Reihenfolge geschieht,

b) in einem zweiten Zeitraum wird der Rührkesselreaktor (6) im Batch-Modus betrieben,

wobei die Abfolge der Schritte a) und b) mindestens einmal wiederholt wird und wobei der erste und/oder zweite Zeitraum sich in ihrer Länge unterscheiden können, wenn die Abfolge der Schritte a) und b) wiederholt wird.

4.  Verfahren nach einem oder mehreren der vorstehenden Ansprüche, wobei der Reaktor (6) gasdicht geschlossen ist und das Verfahren umfasst:

- Messen einer Druckveränderung in einem Kopfraum (22) des Reaktors (6), um eine Aktivität der Bakterien zu überwachen.

5.  Verfahren nach einem oder mehreren der vorstehenden Ansprüche, umfassend

- kontinuierliche oder diskontinuierliche Abgabe von stickstoffhaltigem Gas aus dem Kopfraum (22), wobei der Stickstoff durch anaerobe Ammoniumoxidreaktion entsteht.

6.  Verfahren nach einem oder mehreren der vorstehenden Ansprüche, wobei die Zufuhr wie folgt erfolgt:

- Zufuhr einer ersten Flüssigzufuhr (30), die Nitrit-Ionen umfasst
- Zufuhr einer zweiten Flüssigzufuhr (31), die Ammonium-Ionen umfasst.

7.  Verfahren nach einem oder mehreren der vorstehenden Ansprüche, umfassend

- Zufuhr einer dritten oder weiteren Flüssigzufuhr (32), die Ionen von Alkalimetall und/oder Erdalkalimetall umfasst.

8.  Verfahren nach einem oder mehreren der vorstehenden Ansprüche, ferner umfassend

- Zufuhr von entnommenem Flüssigprodukt aus kontinuierlichem Betrieb oder von mindestens einem von dem Flüssiganteil aus dem Rührkesselreaktor (6) zu einem weiteren Reaktor und
- Kultivieren von Bakterien mittels anaerober Ammoniumoxidation in dem weiteren Reaktor.

9.  Verfahren nach einem oder mehreren der vorstehenden Ansprüche, ferner umfassend

- Isolieren von Bakterien mittels anaerober Ammoniumoxidation aus entnommenem Flüssigprodukt.

**10.** Verfahren nach einem oder mehreren der vorstehenden Ansprüche, ferner umfassend

- Behandeln von ammoniumhaltigem Abwasser mit entnommenem Flüssigprodukt.

**11.** Verfahren nach einem oder mehreren der vorstehenden Ansprüche, ferner umfassend

- Zufuhr von entnommenem Flüssigprodukt zu einem Bioreaktor, der mikrobielle Population(en) enthält.

**Revendications**

**1.** Procédé de culture de bactéries intervenant dans l'oxydation anaérobie de l'ammonium, comprenant les étapes consistant à

- réaliser la culture de bactéries intervenant dans l'oxydation anaérobie de l'ammonium dans un réacteur à cuve agitée (6),
le procédé comprenant en outre les étapes consistant à :

A) introduire des ions nitrites et des ions ammonium dans au moins une charge liquide (30, 31, 32) dans le réacteur à cuve agitée (6),
B) décharger le produit liquide depuis le réacteur à cuve agitée (6),

dans lequel

i) les étapes consistant à introduire en A) et décharger en B) sont effectuées en continu, ou
ii) l'étape consistant à introduire en A) est effectuée de manière répétée et discontinue, et lorsque l'étape consistant à décharger en B) est effectuée, une fraction du contenu liquide dans le réacteur à cuve agitée (6) est déchargée, ce qui est effectué de manière répétée et discontinue,

**caractérisé en ce que** :

- le procédé comprend une étape consistant à dégazer la ou les charges liquides (30, 31, 32), et
- dans la culture, les bactéries sont cultivées en tant que cellules de plancton.

**2.** Procédé selon la revendication 1, dans lequel dans ii) la fraction du contenu liquide est $\leq$ 10 % en volume, préférentiellement $\leq$ 3 % en volume du contenu liquide.

**3.** Procédé selon une ou plusieurs des revendications précédentes, dans lequel dans ii) le fonctionnement du réacteur à cuve agitée (6) est réalisé de la manière suivante :

a) dans une première période, la charge liquide (30, 31, 32) est ajoutée et la fraction liquide est déchargée, ce qui est effectué soit simultanément soit en partie simultanément, soit séquentiellement dans n'importe quel ordre,
b) pendant une seconde période, le réacteur à cuve agitée (6) fonctionne en mode batch,

dans lequel la séquence des étapes a) et b) est répétée au moins une fois et dans lequel la première et/ou la seconde période peuvent varier en longueur lorsque la séquence des étapes a) et b) est répétée.

**4.** Procédé selon une ou plusieurs des revendications précédentes, dans lequel le réacteur (6) est fermé de manière étanche aux gaz et le procédé comprend l'étape consistant à :

- mesurer une variation de pression dans un volume libre (22) du réacteur (6) afin de surveiller l'activité des bactéries.

**5.** Procédé selon une ou plusieurs des revendications précédentes, comprenant l'étape consistant à

- libérer de manière continue ou discontinue un gaz contenant de l'azote depuis le volume libre (22), l'azote étant formé dans la réaction d'oxydation anaérobie de l'ammonium.

**6.** Procédé selon une ou plusieurs des revendications précédentes, dans lequel l'étape consistant à introduire est effectuée de la manière suivante :

- introduire une première charge liquide (30) comprenant des ions nitrite
- introduire une deuxième charge liquide (31) comprenant des ions ammonium.

**7.** Procédé selon une ou plusieurs des revendications précédentes, comprenant l'étape consistant à

- introduire une troisième charge liquide ou une charge supplémentaire (32) comprenant des ions d'un métal alcalin et/ou d'un métal alcalino-terreux.

**8.** Procédé selon une ou plusieurs des revendications précédentes, comprenant en outre l'étape consistant à

- introduire le produit liquide déchargé depuis le fonctionnement continu ou au moins l'une des fractions liquides provenant du réacteur à cuve agitée (6) dans un réacteur supplémentaire et
- cultiver des bactéries intervenant dans l'oxydation anaérobie de l'ammonium dans le réacteur supplémentaire.

**9.** Procédé selon une ou plusieurs des revendications précédentes, comprenant en outre l'étape consistant à

- isoler les bactéries intervenant dans l'oxydation anaérobie de l'ammonium d'avec le produit liquide déchargé.

**10.** Procédé selon une ou plusieurs des revendications précédentes, comprenant en outre l'étape consistant à

- traiter les eaux usées contenant de l'ammonium avec le produit liquide déchargé.

**11.** Procédé selon une ou plusieurs des revendications précédentes, comprenant en outre l'étape consistant à

- introduire le produit liquide déchargé dans un bioréacteur contenant une ou plusieurs populations microbiennes.

Fig.1

EP 3 502 234 B1

Fig. 2

Fig. 3

Fig. 4

Fig. 5

Fig. 6

Fig. 7

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **VAN NIFTRIK et al.** Anaerobic ammonium-oxidizing bacteria: Unique microorganisms with exceptional properties. *Microbiol. Mol. Biol. Rev.*, 2012, vol. 76 (3), 585-596
- **VAN TEESELING et al.** The S-layer protein of the anammox bacterium Kuenenia stuttgartiensis is heavily o-glycosylated. *Front. Microbiol.*, 2016, vol. 7, 1721
- **OSHIKI, M. et al.** Ecology and physiology of anaerobic ammonium oxidizing bacteria. *Environ. Microbiol.*, 2016, vol. 18 (9), 2784-2796
- **KARTAL, B.** ; **KELTJENS, J. T.** Anammox biochemistry: A tale of heme c proteins. *Trends Biochem.Sci.*, 2016, vol. 41 (12), 998-1011
- **DE ALMEIDA et al.** B. Membrane-bound electron transport systems of an anammox bacterium: A complexome analysis. *Biochimica et Biophysica Acta (BBA) - Bioenergetics*, 2016, vol. 1857 (10), 1694-704
- **STROUS, M. et al.** Deciphering the evolution and metabolism of an anammox bacterium from a community genome. *Nature*, 2006, vol. 440 (7085), 790-794
- **VAN DER STAR, W. R. L. et al.** The membrane bioreactor: A novel tool to grow anammox bacteria as free cells. *Biotechnol. Bioeng.*, 2008, vol. 101 (2), 286-294
- anammox-growth physiology, cell biology, and metabolism. **KARTAL, B. et al.** Adv. Microb. Physiol.. Academic Press, 2012, vol. 60, 211-262
- **VAN DER STAR et al.** The membrane bioreactor: A novel tool to grow anammox bacteria as free cells. *Biotechnol. Bioeng.*, 2008, vol. 101 (2), 286-294
- **OKAMOTO, H. et al.** Development of a fixed-bed anammox reactor with high treatment potential. *Biodegradation*, 2013, vol. 24 (1), 99-110
- **TRIGO, C.** Start-up of the anammox process in a membrane bioreactor. *J. Biotechnol.*, 2006, vol. 126 (4), 475-487
- Factors affecting the treatment of reject water by the anammox process. **ZHIGANG LI et al.** Bioresource Technology. Elsevier, 01 March 2011, vol. 102, 5702-5708
- **MAGRÍ ALBERT et al.** Feasibility and interest of the anammox process as treatment alternative for anaerobic digester supernatants in manure processing - An overview. *Journal of Environmental Management*, vol. 131, 170-184
- **LIU YUAN et al.** Upgrading of the symbiosis of Nitrosomanas and anammox bacteria in a novel single-stage partial nitritation-anammox system: Nitrogen removal potential and Microbial characterization. *Bioresource Technology*, 29 July 2017, vol. 244, 463-472
- Implementation of the Anammox Process for Improved Nitrogen Removal. **GÜVEN D et al.** Journal of Environmental Science and Health. Env.Science and Engineer, vol. A39, 1729-1738
- Start-up and stabilization of an Anammox process from a non-acclimatized sludge in CSTR. **SAMIK BAGCHI et al.** Journal of Industrial Microbiology & Biotechnology ; Official Journal of the Society for Industrial Microbiology. Springer, 12 June 2010, vol. 37, 943-952
- **KANDELER, E.** ; **GERBER, H.** Short-term assay of soil urease activity using colorimetric determination of ammonium. *Biol. Fertil. Soils*, 1988, vol. 6 (1), 68-72
- **MARCO-URREA, E et al.** Transformation and carbon isotope fractionation of tetra- and trichloroethene to trans-dichloroethene by Dehalococcoides sp. strain CBDB1. *Environ. Sci. Technol.*, 2011, vol. 45, 1555-1562
- **ADRIAN, L et al.** Growth of Dehalococcoides strains with chlorophenols as electron acceptors. *Environ. Sci. Technol.*, 2007, vol. 41 (7), 2318-2323
- **DAIMS, H. et al.** The domain-specific probe Eub338 is insufficient for the detection of all bacteria: Development and evaluation of a more comprehensive probe set. *Syst. Appl. Microbiol.*, 1999, vol. 22 (3), 434-444
- **SCHMID, M. et al.** Molecular evidence for genus level diversity of bacteria capable of catalyzing anaerobic ammonium oxidation. *Syst. Appl. Microbiol.*, 2000, vol. 23 (1), 93-106
- **KLINDWORTH, A.** ; **PRUESSE, E.** ; **SCHWEER, T.** ; **PEPLIES, J.** ; **QUAST, C.** ; **HORN, M.** ; **GLÖCKNER, F. O.** Evaluation of general 16S ribosomal RNA gene PCR primers for classical and next-generation sequencing-based diversity studies. *Nucleic Acids Res.*, 2013, vol. 41 (1), e1
- **QUAST, C.** ; **PRUESSE, E.** ; **YILMAZ, P.** ; **GERKEN, J.** ; **SCHWEER, T.** ; **YARZA, P.** ; **PEPLIES, J.** ; **GLÖCKNER, F. O.** The SILVA ribosomal RNA gene database project: improved data processing and web-based tools. *Nucleic Acids Res.*, 2013, vol. 41, D590-D596

- **ONDOV, B. D.** ; **BERGMAN, N. H.** ; **PHILLIPPY, A. M.** Interactive metagenomic visualization in a Web browser. *BMC Bioinformatics*, 2011, vol. 12 (1), 385
- **LEE, Z. M. P.** ; **BUSSEMA, C.** ; **SCHMIDT, T. M.** rrnDB: documenting the number of rRNA and tRNA genes in bacteria and archaea. *Nucleic Acids Res.*, 2009, vol. 37, D489-D493
- **STROUS, M.** ; **HEIJNEN, J. J.** ; **KUENEN, J. G.** ; **JETTEN, M. S. M.** The sequencing batch reactor as a powerful tool for the study of slowly growing anaerobic ammonium-oxidizing microorganisms. *Appl. Microbiol. Biotechnol.*, 1998, vol. 50 (5), 589-596
- **STROUS, M. et al.** The sequencing batch reactor as a powerful tool for the study of slowly growing anaerobic ammonium-oxidizing microorganisms. *Appl. Microbiol. Biotechnol.*, 1998, vol. 50 (5), 589-596
- **VAN DER HEIJDEN, R.** ; **HEIJNEN, J.** ; **HELLINGA, C.** ; **ROMEIN, B.** ; **LUYBEN, K.** Linear constraint relations in biochemical reaction systems: I. Classification of the calculability and the balanceability of conversion rates. *Biotechnol. Bioeng.*, 1994, vol. 43 (1), 3-10
- **OSHIKI, M. et al.** Cultivation of planktonic anaerobic ammonium oxidation (anammox) bacteria using membrane bioreactor. *Microbes Environ.*, 2013, vol. 28 (4), 436-443
- **LOTTI, T. et al.** Faster through training: The anammox case. *Water Res.*, 2015, vol. 81, 261-268
- **MAO, N. et al.** Engineering application of anaerobic ammonium oxidation process in wastewater treatment. *World J. Microbiol. Biotechnol.*, 2017, vol. 33 (8), 153
- Chapter four - cultivation, detection, and ecophysiology of anaerobic ammonium-oxidizing bacteria. **KARTAL, B. et al.** Methods Enzymol.. Academic Press, 2011, vol. 486, 89-108
- **LOTTI, T. et al.** Physiological and kinetic characterization of a suspended cell anammox culture. *Water Res.*, 2014, vol. 60, 1-14
- **LOTTI, T et al.** Physiological and kinetic characterization of a suspended cell anammox culture. *Water Res.*, 2014, vol. 60, 1-14
- **LACKNER, S. et al.** Swinging ORP' as operation strategy for stable reject water treatment by nitritation-anammox in sequencing batch reactors. *Chem. Eng. J.*, 2012, vol. 180, 190-196
- **YANG, J. et al.** Oxidation-reduction potential (ORP) as a control parameter in a single-stage partial nitritation/anammox process treating reject water. *J. Chem. Technol. Biotechnol.*, 2015, vol. 91 (10), 2582-2589
- **ZHANG, L.** ; **NARITA, Y.** ; **GAO, L.** ; **ALI, M.** ; **OSHIKI, M.** ; **OKABE, S.** Maximum specific growth rate of anammox bacteria revisited. *Water Res.*, 2017, vol. 116 (1), 296-303
- **LOTTI, T et al.** Faster through training: The anammox case.. *Water Res.*, 2015, vol. 81, 261-268
- cultivation, detection, and ecophysiology of anaerobic ammonium-oxidizing bacteria. **KARTAL, B. et al.** Methods Enzymol.. Academic Press, 2011, vol. 486, 89-108
- **EGLI, K. et al.** Enrichment and characterization of an anammox bacterium from a rotating biological contactor treating ammonium-rich leachate. *Arch. Microbiol.*, 2001, vol. 175 (3), 198-207
- **DAPENA-MORA, A. et al.** Evaluation of activity and inhibition effects on anammox process by batch tests based on the nitrogen gas production. *Enzyme Microbiol. Technol.*, 2007, vol. 40 (4), 859-865
- **RUSS, L et al.** Interactions between anaerobic ammonium and sulfur-oxidizing bacteria in a laboratory scale model system. *Environ. Microbiol.*, 2014, vol. 16 (11), 3487-3498
- **CARVAJAL-ARROYO, J. M. et al.** Inhibition of anaerobic ammonium oxidizing (anammox) enrichment cultures by substrates, metabolites and common wastewater constituents. *Chemosphere*, 2013, vol. 91 (1), 22-27
- **JIN, R.-C. et al.** The effect of sulfide inhibition on the ANAMMOX process. *Water Res.*, 2013, vol. 47 (3), 1459-1469
- **BROOKS, M. H. et al.** Inhibition of existing denitrification enzyme activity by chloramphenicol. *Appl. Environ. Microbiol.*, 1992, vol. 58 (5), 1746-1753
- **DAPENA-MORA, A**. Evaluation of activity and inhibition effects on anammox process by batch tests based on the nitrogen gas production. *Enzyme Microbiol. Technol.*, 2007, vol. 40 (4), 859-865
- **FERNÁNDEZ, I. et al.** Operation of an anammox SBR in the presence of two broad-spectrum antibiotics. *Process Biochemistry*, 2009, vol. 44 (4), 494-498